# EUROPEAN PATENT APPLICATION

(11) **EP 4 129 985 A1**
(43) Date of publication of application: **08.02.2023**
(21) Application number: 21775340.9
(22) Date of filing: 16.03.2021
(51) Int. Cl.: C07D 233/96, C07D 233/66, C07D 233/70, A61K 31/00, A61K 31/4164, A61K 31/4166

(54) **FLUORESCENT DYE, PREPARATION METHOD THEREFOR AND USE THEREOF**

(30) Priority: 23.03.2020 CN 202010207772
(71) Applicant: Fluorescence Diagnosis (shanghai) Biotech Company Ltd., Shanghai 200231 (CN)
(72) Inventor: ZHANG, Dasheng, Shanghai 200231 (CN)
(74) Representative: Bandpay & Greuter
(86) International application number: PCT/CN2021/080926
(87) International publication number: WO 2021/190346

(57) **Abstract**

A fluorescent dye, a preparation method and uses thereof, wherein the fluorescent dye has the advantages of sensitivity and specificity to viscosity response, low background fluorescence and the like, and can also be used as a fluorescence activated and lightened-up probe for fluorescence labeling, quantification or detection of proteins, enzymes or nucleic acids.

## Description

### Technical Field

The present invention relates to the technical field of fluorescent dye, and particularly relates to a fluorescent dye with viscosity responsiveness and low background fluorescence, as well as a preparation method and uses thereof.

### Background

Chromophores of fluorescent proteins are mainly imidazolinone dye, which has good biocompatibility, and is also advantageous in photostability and adjustable fluorescence properties. According to research findings, this kind of dye is a molecular rotor, and its fluorescence property changes with viscosity variation, namely, when excited by light, free-state molecular rotors release excited-state energy in a non-radiation form by means of excited-state intra-molecular twist; and, when the molecules are in a relatively viscous or rigid environment, the excited-state intra-molecular twist is limited, and the excited-state energy is mainly released in a form of radio-luminescence and shows the property of fluorescence enhancement. Therefore, molecular rotors are often used for detecting changes in micro-environments, etc.

At present, intra-molecular twist and luminescence based on restricted molecular rotors are not only used for viscosity detection, but also are widely used in the construction of fluorescent sensors, protein labeling, nucleic acid labeling, etc.; for instance: after DCVJ binds to BSA and other proteins, the excited-state intra-molecular twist of the molecular rotors is restricted, and the excited-state energy is released in a form of radio-luminescence and is manifested as fluorescence activation, and thus can be used for the detection and quantification of target proteins; after imidazolinone and other dyes bind to DNA and RNA, molecular conformation is restricted so that fluorescence is illuminated, and thus can be used for the no-clean labeling and detection of DNA and RNA; with DHBI as the target molecule, aptamer that can specifically bind to DHBI can be obtained through SELEX screening, thereby constructing the fluorescent aptamer, overcoming the difficulty of lacking natural fluorescent RNA and making it possible to construct fluorescent RNA; with peacock green as the target molecule, a single-chain antibody that can specifically bind to peacock green derivatives and activate fluorescence is obtained by means of phage display, and can be used for labeling of cell membrane proteins; BODIPY and other molecular rotors can bind to amyloid and tua proteins and light up fluorescence, and thus can be used for researches of diseases such as Alzheimer's and Parkinson's.

The types of imidazolinone molecular rotors completely derived from fluorescent protein chromophores are quite limited. Thanks to modification by means of organic synthesis, the spectral range of imidazolinone is significantly expanded, and other properties, such as light stability, oil-water distribution coefficient, etc. can be effective adjusted. However, molecular rotors obtained by this method generally have the disadvantage of high fluorescence background, which causes low detection signal-to-noise ratio and makes it difficult to detect and label samples with small sample sizes, complex components and low substrate abundance, so it is necessary to develop a kind of imidazolinone molecular rotors with low background fluorescence, so as to further expand uses of such molecular rotors.

### Summary of the Invention

The object of the present invention is to provide a fluorescent dye with viscosity responsiveness and low background fluorescence, wherein the viscosity responsiveness of this kind of molecular rotors is that a ratio of fluorescence intensity in glycerol to that in methanol is greater than 10 at a condition of 10⁻⁵ mol.

For one aspect, the present invention provides a fluorescent dye, which is shown as Formula (I), 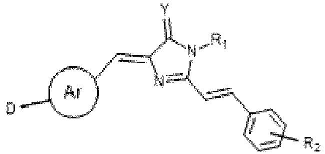 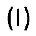 wherein:
Ar is arylene or heteroarylene, and optionally, hydrogen atoms in Ar are independently substituted by halogen atoms; D- is HO- or N(X₁)(X₂)-, and X₁ and X₂ are independently selected from hydrogen, alkyl or modified alkyl; X₁ and X₂ are optionally interconnected, and form an alicyclic heterocycle with N atoms; and X₁ and X₂ are optionally and independently form an alicyclic heterocycle with Ar;
Y is O or S;
R₁ is hydrogen or alkyl; and
R₂ is a halogen atom, -OH or -CN;
wherein:
   the "alkyl" is C₁-C₁₀ straight or branched alkyl; optionally, the "alkyl" is C₁-C₆ straight or branched alkyl; optionally, the "alkyl" is C₁-C₄ straight or branched alkyl; optionally, the "alkyl" is selected from methyl, ethyl, n-propyl, isopropyl, n-butyl, isobutyl, tertiary butyl, sec-butyl, n-amyl, 1-methyl butyl, 2-methyl butyl, 3-methyl butyl, isoamyl, 1-ethyl propyl, neoamyl, n-hexyl, 1-methyl amyl, 2-methyl amyl, 3-methyl amyl, isohesyl, 1,1-dimethyl butyl, 2,2-dimethyl butyl, 3,3-dimethyl butyl, 1,2-dimethyl butyl, 1,3-dimethyl butyl, 2,3-dimethyl butyl, 2-ethyl butyl, n-heptyl, 2-methyl hexyl, 3-methyl hexyl, 2,2-dimethyl amyl, 3,3 dimethyl amyl, 2,3-dimethyl amyl, 2,4-dimethyl amyl, 3-ethyl amyl or 2,2,3-methyl butyl;
   the "modified alkyl" is independently a group obtained by replacing any carbon atom in C₁-C₁₆ straight or branched alkyl with one or more groups selected from halogen atom, -OH, -CO-, -O-, -CN, -SO₃H-, primary amino group, secondary amino group and tertiary amino group, or the carbon-carbon single bond is optionally and independently replaced by a carbon-carbon double bond or a carbon-carbon triple bond;
   the replacement of carbon atoms refers to that carbon atoms or the carbon atoms and hydrogen atoms thereon together are replaced by a corresponding group;
   the "halogen atom" is independently F, Cl, Br or I;
   the " alicyclic heterocycle" is a saturated or unsaturated 4- to 15-membered monocyclic or polycyclic alicyclic heterocycle containing one or more heteroatoms of N, O, S or Si on the ring, and the alicyclic heterocycle containing S heteroatom(s) on the ring include the ones that contain -S-, -SO- or -SO₂- on the ring; the alicyclic heterocycle is optionally substituted by a halogen atom, an alkyl, an aryl or a modified alkyl;
   the "arylene" is independently a 5- to 13-membered (optionally 6- or 10-membered) monocyclic or dicyclic or fused dicyclic or fused polycyclic subaromatic group;
   the "heteroarylene" is independently a 5- to 13-membered (optionally 6- or 10-membered) monocyclic or dicyclic or fused dicyclic or fused polycyclic sub-heteroaromatic group containing one or more heteroatoms selected from N, O, S or Si on the ring;
   the "primary amino group" is R'NH₂ group;
   the "secondary amino group" is R'NHR" group;
   the "tertiary amino group" is R'NR"R‴ group;
   each R', R", R‴ is independently a single bond, hydrogen, alkyl, or alkylene;
   the "alkylene" is C₁-C₁₀ straight or branched alkylene; optionally, it is C₁-C₇ straight or branched alkylene; optionally, it is C₁-C₅ straight or branched alkylene;
   optionally, the "modified alkylene" is a group containing one or more groups selected from -OH, -O-, -NH₂, ethylene glycol unit (-(CH₂CH₂O)ₙ-), -CN -O-CO-, -NH-CO-, -SO₂-O-, -SO-, Me₂N-, Et₂N-, -CH=CH-, -C≡CH, F, Cl, Br, I, and cyano group; and
   optionally, Ar is a structure selected from the following Formulae (II-1) to (II-7):

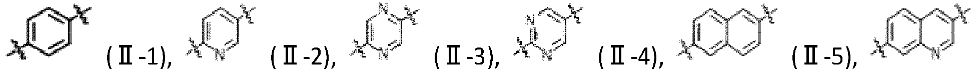

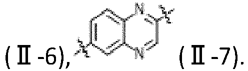

Optionally, the compound represented by Formula (I) is selected from the compounds below:

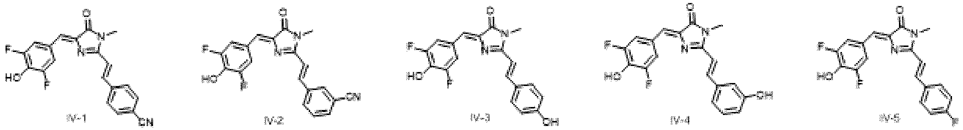

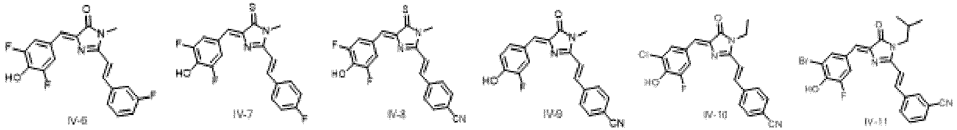

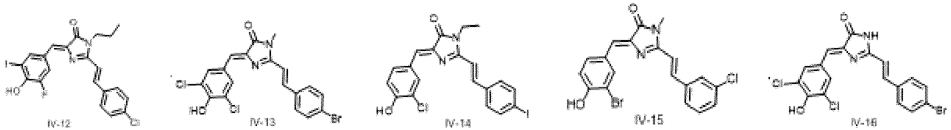

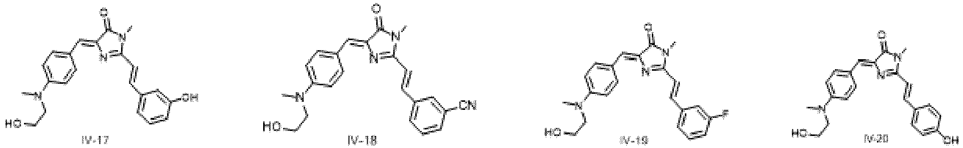

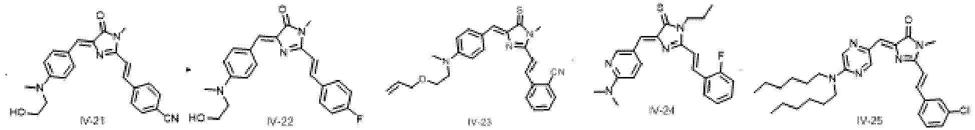

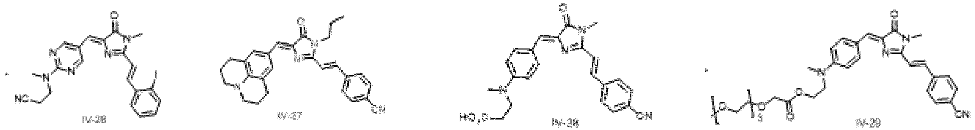

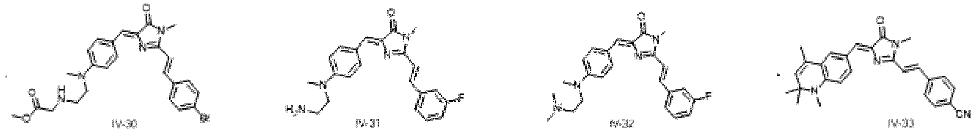

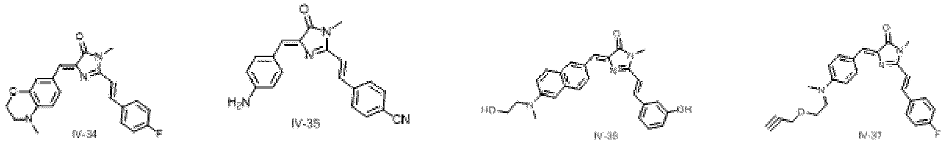

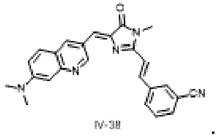

A second aspect of the present invention is to provide a method for preparing the afore-mentioned fluorescent dye, including a step of aldol condensation reaction between a compound of Formula (a) and a compound of Formula (b):

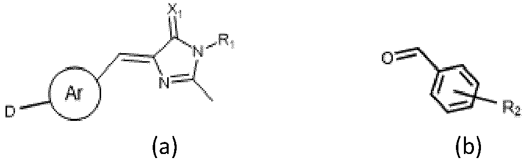

A third aspect of the present invention is to provide uses of the afore-mentioned fluorescent dye in viscosity testing, protein fluorescent labeling, nucleic acid fluorescent labeling, protein quantification or detection, or nucleic acid quantification or detection, wherein the uses are those other than for diagnostic methods of diseases.

A fourth aspect of the present invention is to provide uses of the afore-mentioned fluorescent dye in preparing reagents for viscosity testing, protein fluorescent labeling, nucleic acid fluorescent labeling, protein quantification or detection, or nucleic acid quantification or detection.

A fifth aspect of the present invention is to provide a fluorescent activated and lighted-up probe, comprising the afore-mentioned fluorescent dye.

A sixth aspect of the present invention is to provide uses of the afore-mentioned fluorescent activated and lighted-up probe in protein fluorescent labeling, nucleic acid fluorescent labeling, protein quantification or detection, or nucleic acid quantification or detection, wherein the uses are those other than for diagnostic methods of diseases.

A seventh aspect of the present invention is to provide uses of the afore-mentioned fluorescent activated and lighted-up probe in preparing reagents for protein fluorescent labeling, nucleic acid fluorescent labeling, protein quantification or detection, or nucleic acid quantification or detection.

The fluorescent dye of the present invention can be used for measuring viscosity of samples, such as for the tests of micro-viscosity. According to the embodiments of another aspect, the obtained fluorescent dye can specifically bind to corresponding antibody, aptamer or amyloid, or bind to the protein tag or enzyme via a ligand or inhibitor, thereby obtaining a series of fluorescent activated and lighted-up probes used for fluorescent labeling, quantification or monitoring of proteins, enzymes or nucleic acids.

### Description of Drawings

Fig. 1 is a diagram showing the fluorescence emission intensity at different viscosity conditions of the molecular rotor IV-1 (1×10⁻⁵ M);
Fig. 2 is a diagram showing the fluorescence emission intensity at different viscosity conditions of the molecular rotor IV-2 (1×10⁻⁵ M);
Fig. 3 is a diagram showing the fluorescence emission intensity at different viscosity conditions of the molecular rotor IV-3 (1×10⁻⁵ M);
Fig. 4 is a diagram showing the fluorescence emission intensity at different viscosity conditions of the molecular rotor IV-4 (1×10⁻⁵ M);
Fig. 5 is a diagram showing the fluorescence emission intensity at different viscosity conditions of the molecular rotor IV-5 (1×10⁻⁵ M);
Fig. 6 is a diagram showing the fluorescence emission intensity at different viscosity conditions of the molecular rotor IV-6 (1×10⁻⁵ M);
Fig. 7 is a diagram showing the fluorescence emission intensity at different viscosity conditions of the molecular rotor IV-17 (1×10⁻⁵ M);
Fig. 8 is a diagram showing the fluorescence emission intensity at different viscosity conditions of the molecular rotor IV-18 (1×10⁻⁵ M);
Fig. 9 is a diagram showing the fluorescence emission intensity at different viscosity conditions of the molecular rotor IV-19 (1×10⁻⁵ M);
Fig. 10 is a diagram showing the fluorescence emission intensity at different viscosity conditions of the molecular rotor IV-20 (1×10⁻⁵ M);
Fig. 11 is a diagram showing the fluorescence emission intensity at different viscosity conditions of the molecular rotor IV-21 (1×10⁻⁵ M);
Fig. 12 is a diagram showing the fluorescence emission intensity at different viscosity conditions of the molecular rotor IV-22 (1×10⁻⁵ M);
Fig. 13 is a diagram showing the fluorescence background contrast of molecular rotors IV-39, IV-40, IV-44 and IV-1, IV-2, IV-3, IV-4, IV-5, IV-6, IV-7, IV-8 (1×10⁻⁶ M) in PBS;
Fig. 14 is a diagram showing the fluorescence background contrast of molecular rotors IV-41, IV-43 and IV-17, IV-18, IV-19, IV-20, IV-21, IV-22 (1×10⁻⁶ M) in PBS;
Fig. 15 is a diagram showing the fluorescence background contrast of molecular rotors IV-42 and IV-36 (1×10⁻⁶ M) in PBS;
Fig. 16 is a diagram showing the fluorescence background contrast of molecular rotors IV-45, IV-46 and IV-3 (1×10⁻⁶ M) in PBS;
Fig. 17 is a diagram showing the fluorescence background contrast of molecular rotors IV-47, IV-48 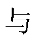 IV-20 (1×10⁻⁶ M) in PBS;
Fig. 18 is a diagram showing the fluorescence background contrast of molecular rotors IV-49, IV-50 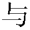 IV-5 (1×10⁻⁶ M) in PBS;
Fig. 19 is a diagram showing the fluorescence background contrast of molecular rotors IV-51 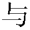 IV-1 (1×10⁻⁶ M) in PBS;
Fig. 20 is the application of molecular rotors IV-1, IV-2, IV-3, IV-4, IV-5, IV-6, IV-17, IV-18, IV-19, IV-20, IV-21, IV-22 in labeling intracellular RNA aptamer, wherein A are cells expressing the target RNA aptamer, and B are cells not expressing the target RNA aptamer;
Fig. 21 is a flow detection diagram for molecular rotors IV-21 and IV-41 for labeling mRNA.

### Embodiments

### Examples:

### Example1:

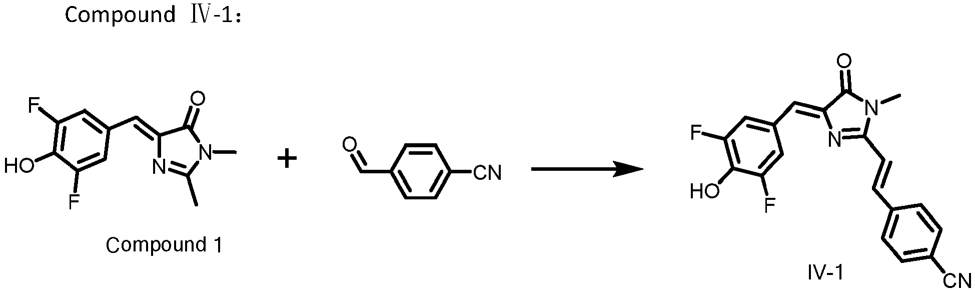

To a stirred solution of Compound 1 (0.504 g, 2 mmol), anhydrous zinc chloride (0.545 g, 4 mmol) in 100 mL THF, 4-Cyanobenzaldehyde (0.626 g, 5 mmol) was added. The complete solution were stirred at 80°C under Ar atomophere. The progress of reaction was monitored on silica gel TLC. After completion of reaction, the solution allowing the reaction to cool to room temperature. The solvent is evaporated to dryness, to give a crude product, then purified by silica gel column chromatography to afford a target compound (0.292 g, 40%).¹H NMR (400 MHz, DMSO-d₆) δ 11.07 (s, 1H), 8.10 (d, J = 8.5 Hz, 2H), 8.06 (dd, J = 7.8, 2.1 Hz, 2H), 8.03 (s, 1H), 7.94 (d, J = 8.3 Hz, 2H), 7.44 (d, J = 15.9 Hz, 1H), 7.03 (s, 1H), 3.29 (s, 3H).MS(ESI): m/z Calcd. For C₂₀H₁₃F₂N₃O₂ 365.0976; found 364.0902, [M-H]⁻.

### Example 2:

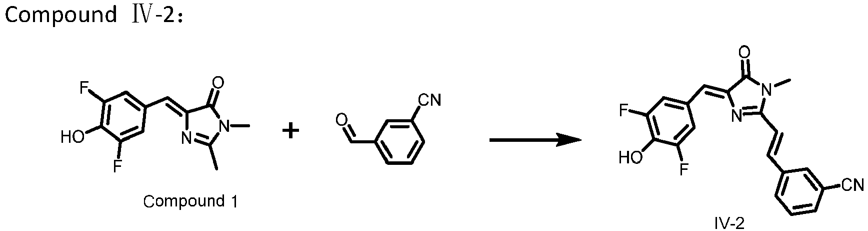

This compound was obtained by following the general procedure for Compound IV -1, (0.475 g, 65%) _{∘} ¹H NMR (400 MHz, DMSO-*d*₆) δ 11.04 (s, 1H), 8.49 (d, *J* = 1.9 Hz, 1H), 8.21 (dt, *J* = 8.0, 1.4 Hz, 1H), 8.10-8.02 (m, 2H), 8.00 (s, 1H), 7.88 (dt, *J* = 7.7, 1.4 Hz, 1H), 7.67 (t, *J* = 7.8 Hz, 1H), 7.43 (d, *J* = 16.0 Hz, 1H), 7.01 (s, 1H), 3.29 (s, 3H).MS(ESI): m/z Calcd. For C₂₀H₁₃F₂N₃O₂ 365.0976; found 364.0903, [M-H]⁻.

### Example 3:

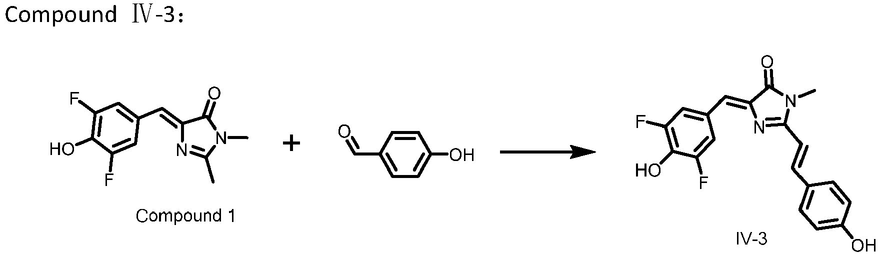

This compound was obtained by following the general procedure for CompoundIV-1, (0.221 g, 31%) _{∘} ¹H NMR (400 MHz, DMSO-d₆) δ 10.94 (s, 1H), 10.11 (s, 1H), 8.07-8.01 (m, 2H), 7.95 (d, J = 15.7 Hz, 1H), 7.74 (d, J = 8.7 Hz, 2H), 7.01 (d, J = 15.7 Hz, 1H), 6.90 (s, 1H), 6.87 - 6.83 (m, 2H), 3.26 (s, 3H).MS(ESI): m/z Calcd. For C₁₉H₁₄F₂N₂O₃ 356.0972; found 355.0901, [M-H]⁻.

### Example 4:

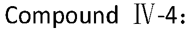

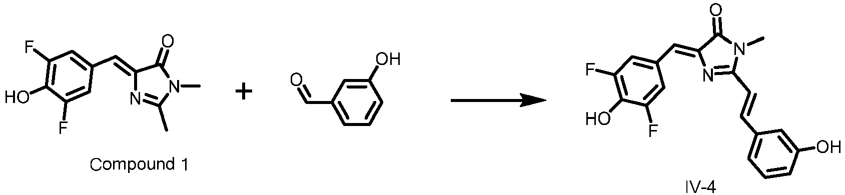

This compound was obtained by following the general procedure for Compound IV -1, (0.207 g, 29%) _{∘} ¹H NMR (400 MHz, DMSO-*d*₆) δ 9.70 (s, 1H), 8.05 (d, *J* = 8.9 Hz, 2H), 7.98 -7.89 (m, 1H), 7.30 (t, *J* = 6.2 Hz, 1H), 7.26 (d, *J* = 8.9 Hz, 1H), 7.15 (d, *J* = 15.8 Hz, 1H), 6.97 (s, 1H), 6.87 (d, *J* = 7.5 Hz, 1H), 3.27 (s, 3H).MS(ESI): m/z Calcd. For C₁₉H₁₄F₂N₂O₃ 356.0972; found 355.0900, [M-H]⁻.

### Example 5:

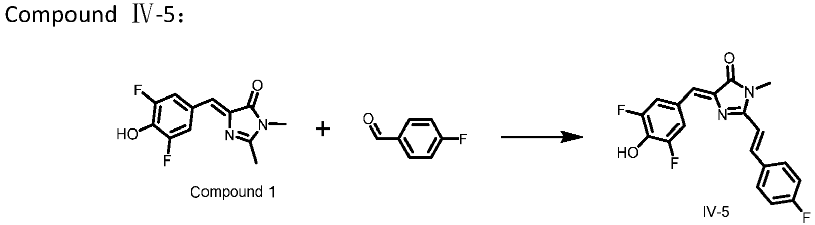

This compound was obtained by following the general procedure for CompoundIV-1, (0.186 g, 26%) _{∘} ¹H NMR (400 MHz, DMSO-*d*₆) δ 8.16 (d, *J* = 8.5 Hz, 2H), 7.99-7.86 (m, 3H), 7.31 (t, *J* = 8.9 Hz, 2H), 7.18 (d, *J* = 15.9 Hz, 1H), 6.95 (s, 1H), 6.83-6.72 (m, 2H), 3.59 (t, *J* = 5.9 Hz, 2H), 3.51 (t, *J* = 5.9 Hz, 2H), 3.27 (s, 3H), 3.05 (s, 3H).MS(ESI): m/z Calcd. For C₁₉H₁₃F₃N₂O₂ 358.0929; found 357.0856, [M-H]⁻.

### Example 6:

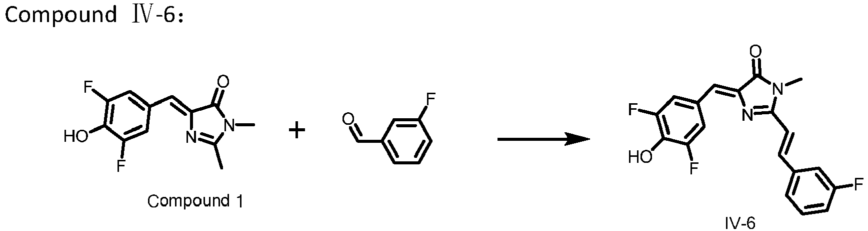

This compound was obtained by following the general procedure for CompoundIV-1, (0.222 g, 31%) _{∘} ¹H NMR (400 MHz, DMSO-*d*₆) δ 11.02 (s, 1H), 8.09-8.03 (m, 2H), 8.01 (d, *J* = 15.8 Hz, 1H), 7.85 (dt, *J* = 10.6, 2.1 Hz, 1H), 7.72 (d, *J* = 7.8 Hz, 1H), 7.51 (td, *J* = 8.0, 6.1 Hz, 1H), 7.34 (d, *J* = 15.9 Hz, 1H), 7.28 (td, *J* = 8.7, 2.7 Hz, 1H), 7.00 (s, 1H), 3.28 (s, 3H).MS(ESI): m/z Calcd. For C₁₉H₁₃F₃N₂O₂ 358.0929; found 357.0857, [M-H]⁻.

### Example 7:

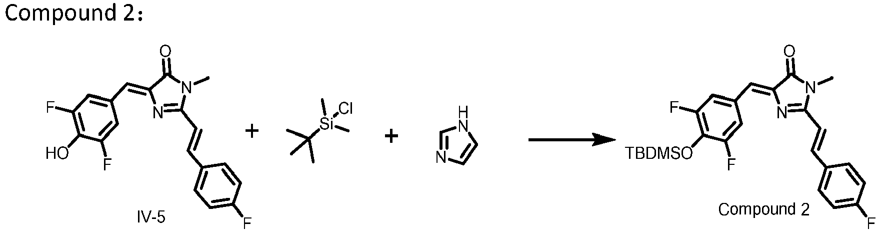

To a stirred solution of compound IV-5 (0.716 g, 2.0 mmol) , TBSCI (0.450 g, 3.0 mmol), in 50 mL dry DMF, and imidazole (0.204 g, 3.0 mmol) was added. The complet solution was stirred for 3 hours at room temperature under Ar atmosphere. The mixture was poured into 150 mL water and extracted with DCM. The organic layer was dried over anhydrous MgSO₄, filtered, and concentrated under reduced pressure to give the crude product, then purified by silica gel column chromatography to afford the compound 2 (0.927 g, 98%).¹H NMR (400 MHz, DMSO-*d*₆) δ 8.16 (d, *J* = 8.5 Hz, 2H), 7.99-7.86 (m, 3H), 7.31 (t, *J* = 8.9 Hz, 2H), 7.18 (d, *J* = 15.9 Hz, 1H), 6.95 (s, 1H), 6.83-6.72 (m, 2H), 3.59 (t, *J =* 5.9 Hz, 2H), 3.51 (t, *J* = 5.9 Hz, 2H), 3.27 (s, 3H), 3.05 (s, 3H), 1.50(s, 9 H), 0.2(s, 6 H).MS(ESI): m/z Calcd. For C₂₅H₂₈F₃N₂O₂Si 473.2; found 473.2, [M+H]⁺.

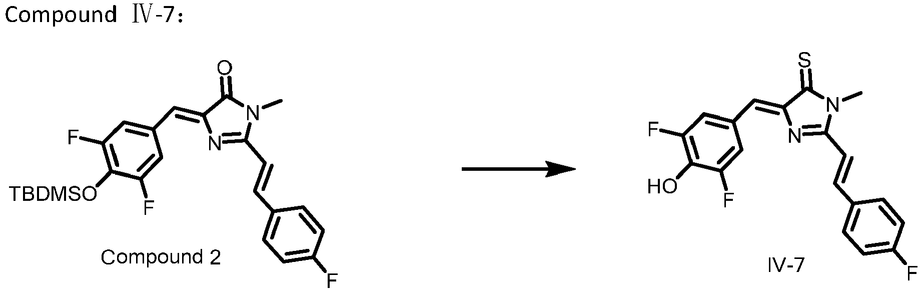

Compound 2 (0.473 g, 1 mmol), Lawesson's reagent (0.808 g, 2 mmol) was in 250 mL three neck bottles and dissoved in 100 ml toluene. Two drops of aniline was added. The reaction mixture was reflexed until the TLC showed the complete the reaction. The solvent was removed under reduce pressure to give the crude product which was redissoved in 50 mL DCM, then TBAF (0.313 g, 1.2 mmol) was added. The mixture was stirred at rt under Ar atomophere. After complete the reaction, the solvent was removed under reduce pressure to give the crude product, then purified by silica gel column chromatography to afford a target compound IV-7 (0.209 g, 56%). ¹H NMR (400 MHz, DMSO-*d*₆) δ 8.17 (d, *J* = 8.5 Hz, 2H), 7.98-7.86 (m, 3H), 7.31 (t, *J* = 8.9 Hz, 2H), 7.18 (d, *J* = 15.9 Hz, 1H), 6.95 (s, 1H), 6.83-6.72 (m, 2H), 3.59 (t, *J* = 5.9 Hz, 2H), 3.51 (t, *J* = 5.9 Hz, 2H), 3.27 (s, 3H), 3.05 (s, 3H).MS(ESI): m/z Calcd. For C₁₉H₁₃F₃N₂NaOS 397.0598; found 397.0597, [M+Na]⁺.

### Example 8:

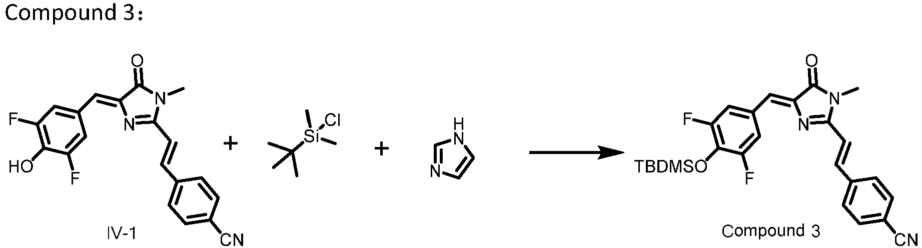

This compound was obtained by following the general procedure for CompoundIV -1Compound 2, (0.932 g, 99%) _{∘} ¹H NMR (400 MHz, DMSO-d₆) δ 8.10 (d, J = 8.5 Hz, 2H), 8.06 (dd, J = 7.8, 2.1 Hz, 2H), 8.03 (s, 1H), 7.94 (d, J = 8.3 Hz, 2H), 7.44 (d, J = 15.9 Hz, 1H), 7.03 (s, 1H) , 3.29 (s, 3H), 1.51 (s, 9H), 0.29 (s, 9H).MS(ESI): m/z Calcd. For C₂₆H₂₃F₂N₃O₂Si 480.2; found 480.2, [M+H]⁺.

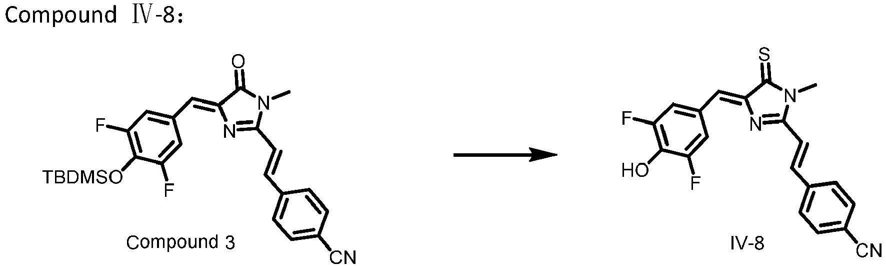

This compound was obtained by following the general procedure for Compound IV-7, (0.332 g, 49%) _{∘} ¹H NMR (400 MHz, DMSO-d₆) δ 11.00 (s, 1H), 8.11 (d, J = 8.5 Hz, 2H), 8.07 (dd, J = 7.8, 2.1 Hz, 2H), 8.04 (s, 1H), 7.94 (d, J = 8.3 Hz, 2H), 7.44 (d, J = 15.9 Hz, 1H), 7.03 (s, 1H), 3.29 (s, 3H).HMS(ESI): m/z Calcd. For C₂₀H₁₃F₂N₃NaOS 404.0645; found 404.0646, [M+Na] ⁺.

### Example 9:

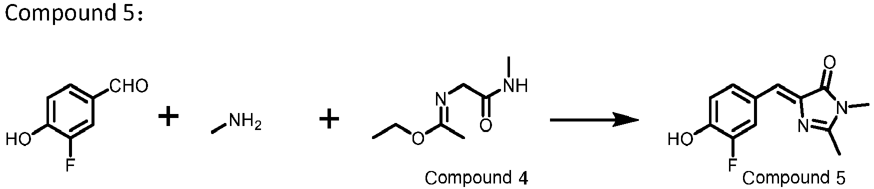

To a stirred solution of 3-Fluoro-4-hydroxybenzaldehyde (0.560 g, 4.0 mmol) and 5 mL 33% methylamine aqueous solution in 40 mL anhydrous ethanol, 10 g Na₂SO₄ was added in one portion. The obtained mixture was stirred and kept at room temperature for 24 hr, then filtered and dried with additional Na₂SO₄. The solvent was removed under reduce pressure to give the intermediate which was used directly without any further purification. After re-dissolved in 10 mL anhydrous methanol, compound 4 (0.790 g, 5.0 mmol) was added. The complex was stirred and kept at room temperature for 12 hr, the precipitated product was filtered and washed with cooled methanol for three times to give the yellow compound 5.
(0.796 g, 85%).¹H NMR (400 MHz, DMSO-d₆) δ10.52 (s, 1H), 8.19 (m, 1H), 7.76 (m, 1H), 6.99 (t, J=8.8 Hz, 1H), 6.89 (s, 1H), 3.09 (s, 3H), 2.34(s, 3H). MS(ESI): m/z Calcd. For C₁₂H₁₀FN₂O₂ 234.2; found 234.2, [M-H]⁻.

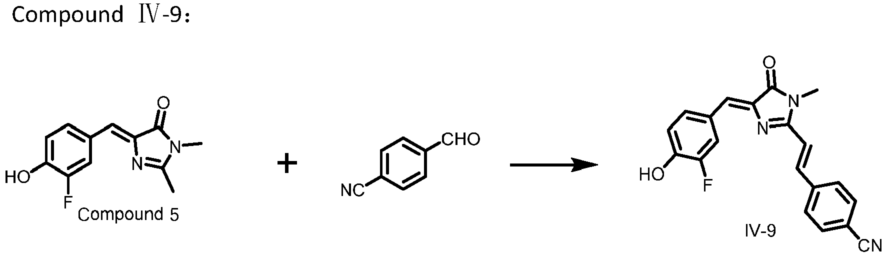

This compound was obtained by following the general procedure for Compound IV -1, (0.239 g, 21%) _{∘} ¹H NMR (400 MHz, DMSO-d₆) δ10.52 (s, 1H), 8.11 (d, J = 8.5 Hz, 2H), 8.07 (d, J = 7.8 Hz, 2H), 7.84 (d, J=8.0 Hz, 1 H), 7.67 (d, J=8.4 Hz, 1 H),7.32 (m, 1 H), 6.99 (t, J=8.8 Hz, 1H), 6.89 (s, 1H), 6.78 (m, 1H), 2.34(s, 3H). HR-MS(ESI): m/z Calcd. For C₂₀H₁₃FN₃O₂ 346.0997; found 346.0998, [M-H]⁻.

### Example 10:

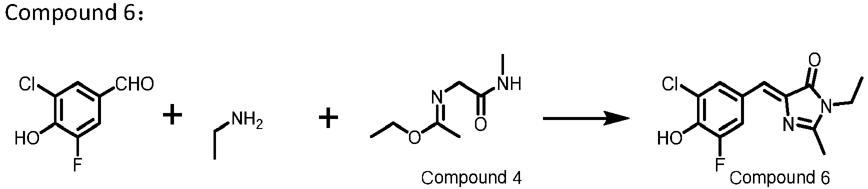

This compound was obtained by following the general procedure for Compound 5, (0.812 g, 91%) _{∘} ¹H NMR (400 MHz, CD₃OD) δ 7.28 (s, 1H), 7.19 (d, J = 8.0 Hz, 2H), 3.59 (t, J=5.6Hz, 3H), 3.12 (s, 3H), 1.23(q, J=5.6Hz, 3H). MS(ESI): m/z Calcd. For C₁₃H₁₁ClFN₂O₂ 281.0; found 281.0,
[M-H]⁻.

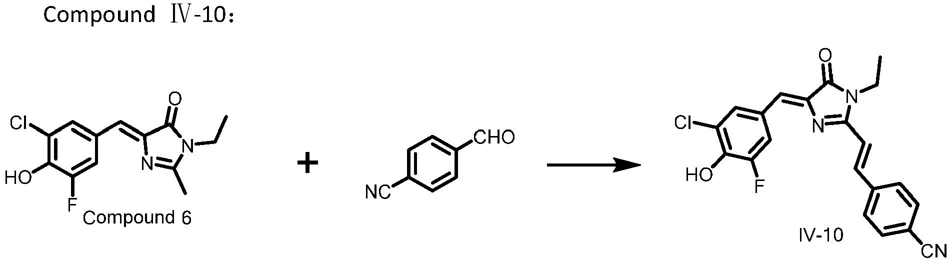

This compound was obtained by following the general procedure for Compound IV -1, (0.239 g, 21%)_{∘}¹H NMR (400 MHz, CD₃OD) δ 7.84 (s, 2H), 7.28 (s, 1H), 7.19 (d, J = 8.0 Hz, 2H), 3.12 (s, 3H), 1.23(q, J=5.6Hz, 3H). HR-MS(ESI): m/z Calcd. For C₂₁H₁₄ClFN₃O₂ 395.0837; found394.0764, [M-H]⁻.

### Example 11:

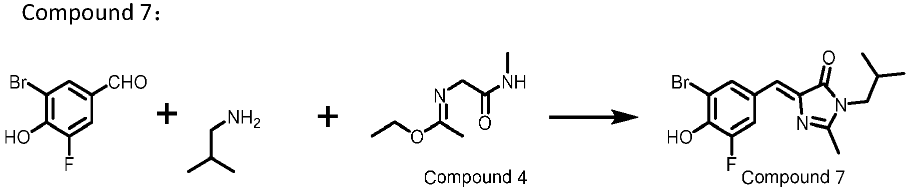

This compound was obtained by following the general procedure for Compound 5, (0.812 g, 91%) _{∘} ¹H NMR (400 MHz, CD₃OD) δ 7.28 (s, 1H), 7.19 (d, J = 8.0 Hz, 2H), 3.81 (s, 3H), 3.12 (s, 3H), 3.12 (s, 3H) , 1.58(m, 1H), 1.11 (d, 6H). MS(ESI): m/z Calcd. For C₁₅H₁₅BrFN₂O₂ 353.0; found 3.0, [M-H]⁻.

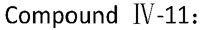

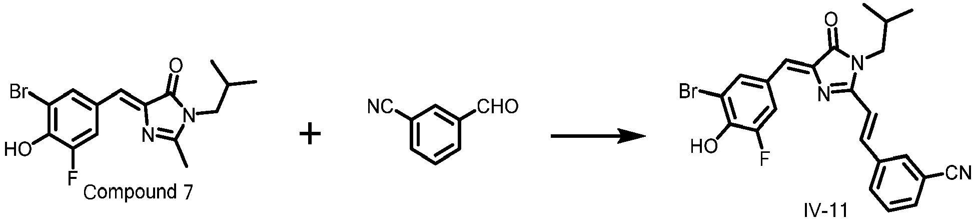

This compound was obtained by following the general procedure for Compound IV -1, (0.209 g, 22%) _{∘} ¹H NMR (400 MHz, CD₃OD) δ 8.15 (d, J = 8.8 Hz, 2H), 7.95 (d, J = 15.7 Hz, 1H), 7.74 (d, J = 8.7 Hz, 2H), 7.28 (s, 1H), 7.19 (d, J = 8.0 Hz, 2H),7.01 (d, J = 15.7 Hz, 1H), 6.87 - 6.83 (m, 2H), 3.12 (s, 3H) , 1.58(m, 1H), 1.11 (d, 6H). HR-MS(ESI): m/z Calcd. For C₂₃H₁₉BrFN₃O₂ 467.0645; found 466.0572, [M-H]⁻.

### Example 12:

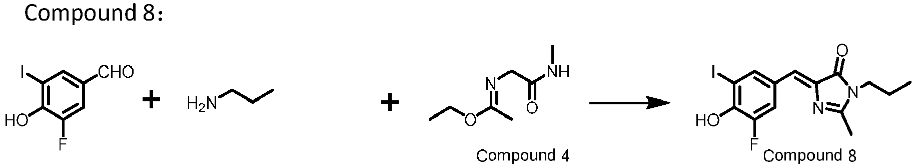

This compound was obtained by following the general procedure for Compound 5, (0.812 g, 91%) _{∘} ¹H NMR (400 MHz, CD3OD) δ10.52 (s, 1H), 7.76 (d, J=8.5 Hz, 2H), 6.95 (s, 1H), 3.79 (t, J=5.2 Hz, 2H), 3.35 (s, 3H), 2.39(t, J=4.8 Hz, 2H), 1.40 (m, 2H), 1.20(t, J=4.8 Hz, 3H). MS(ESI): m/z Calcd. For C₁₄H₂₁₄FIN₂O₂ 388.0; found 388.0, [M-H]⁻.

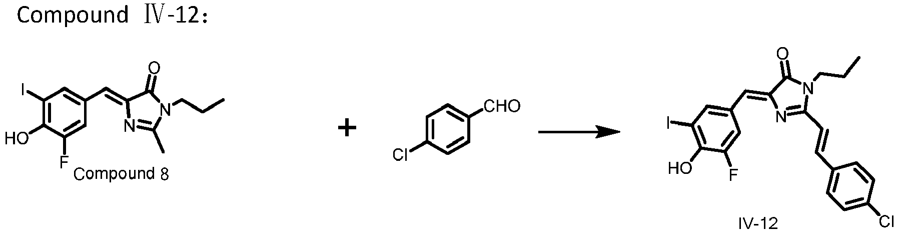

This compound was obtained by following the general procedure for Compound IV -1, (0.156 g, 18%) _{∘} ¹H NMR (400 MHz, CD₃OD) δ10.52 (s, 1H), 7.98-7.86 (m, 3H), 7.76 (d, J=8.5 Hz, 2H), 7.31 (t, *J* = 8.9 Hz, 2H), 7.18 (d, *J* = 15.9 Hz, 1H), 6.95 (s, 1H), 3.79 (t, J=5.2 Hz, 2H), 3.35 (s, 3H), 2.39(t, J=4.8 Hz, 2H), 1.40 (m, 2H), 1.20(t, J=4.8 Hz, 3H). HR-MS(ESI): m/z Calcd. For C₂₁H₁₇ClFIN₂O₂ 510.0007; found408.9936, [M-H]⁻.

### Example 13:

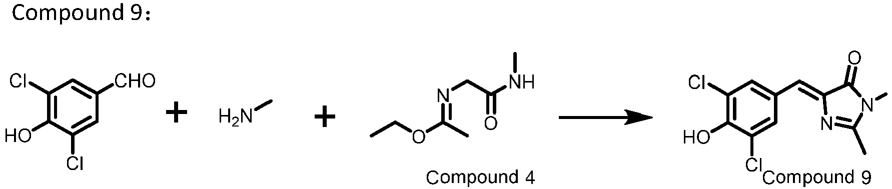

This compound was obtained by following the general procedure for Compound 5, (0.732 g, 93%) _{∘} ¹H NMR (400 MHz, CD₃OD) δ10.52 (s, 1H), 7.76 (d, J=8.5 Hz, 2H), 6.95 (s, 1H), 3.10 (s, 3H), 2.39(s, 3H). MS(ESI): m/z Calcd. For C₁₂H₁₀Cl₂N₂O₃ 284.0; found 283.0, [M-H]⁻.

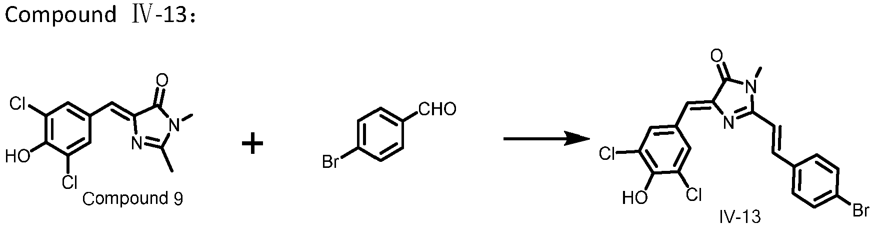

This compound was obtained by following the general procedure for Compound IV -1, (0.156 g, 18%) _{∘} ¹H NMR (400 MHz, CD₃OD) δ10.52 (s, 1H), 8.07 - 8.01 (m, 2H), 7.95 (d, J = 15.7 Hz, 1H),7.76 (d, J=8.5 Hz, 2H), 7.01 (d, J = 15.7 Hz, 1H),6.95 (s, 1H), 6.87 - 6.83 (m, 2H), 3.10 (s, 3H), 2.39(s, 3H).HR-MS(ESI): m/z Calcd. For C₁₉H₁₃BrCl₂N₂O₂ 449.9537; found 448.9455,
[M-H]⁻.

### Example 14:

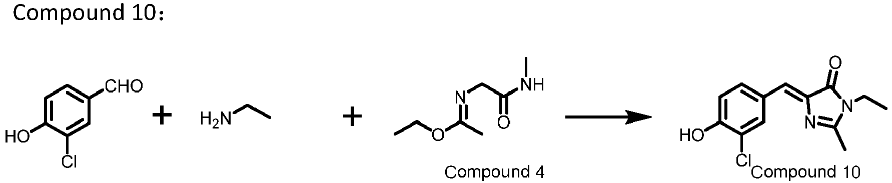

This compound was obtained by following the general procedure for Compound 5, (0.732 g, 93%) _{∘} ¹H NMR (400 MHz, DMSO-d₆) δ10.52 (s, 1H), 8.19 (m, 1H), 7.76 (m, 1H), 6.99 (t, J=8.8 Hz, 1H), 6.89 (s, 1H), 3.01 (q, J=4.8 Hz, 2H), 2.34(s, 3H), 1.21 (t, J=4.8 Hz, 3H). MS(ESI): m/z Calcd. For C₁₃H₁₃ClN₂O₂ 264.1; found 263.1, [M-H]⁻.

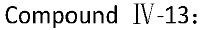

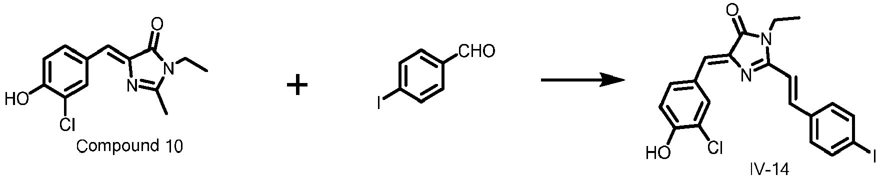

This compound was obtained by following the general procedure for CompoundIV-1, (0.156 g, 18%) _{∘} ¹H NMR (400 MHz, DMSO-d₆) δ10.52 (s, 1H), 8.19 (m, 1H), 8.07 - 8.01 (m, 2H), 7.95 (d, J = 15.7 Hz, 1H), 7.76 (m, 1H), 7.01 (d, J = 15.7 Hz, 1H), 6.99 (t, J=8.8 Hz, 1H), 6.89 (s, 1H), 6.87 - 6.83 (m, 2H), 3.01 (q, J=4.8 Hz, 2H), 2.34(s, 3H), 1.21 (t, J=4.8 Hz, 3H). HR-MS(ESI): m/z Calcd. For C₂₁H₁₃ClIN₂O₂ 486.9716; found 486.9715, [M-H]⁻.

### Example 15:

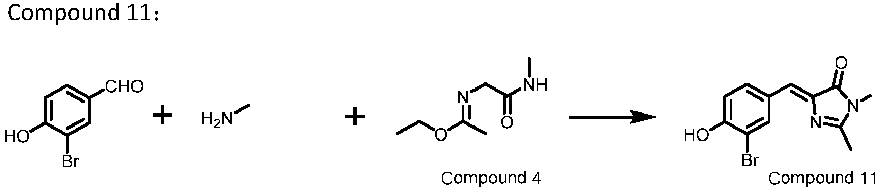

This compound was obtained by following the general procedure for Compound 5, (0.732 g, 93%) _{∘} ¹H NMR (400 MHz, CD₃OD) δ10.52 (s, 1H), 8.19 (m, 1H), 7.76 (m, 1H), 6.99 (t, J=8.8 Hz, 1H), 6.89 (s, 1H), 3.05 (s, 3H), 2.34(s, 3H). MS(ESI): m/z Calcd. For C₁₂H₁₁BrN₂O₂ 294.0; found 293.0, [M-H]⁻.

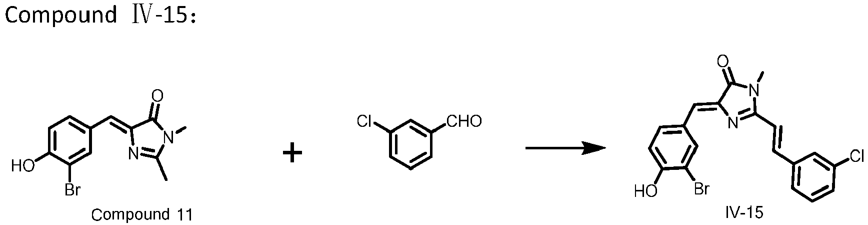

This compound was obtained by following the general procedure for Compound IV -1, (0.156 g, 18%) _{∘} ¹H NMR (400 MHz, CD3OD) δ10.52 (s, 1H), 8.19 (m, 1H), 7.95 (d, J = 15.7 Hz, 1H), 7.76 (m, 1H), 7.49(m, 1H), 7.40-7.22(m, 3H), 7.01 (d, J = 15.7 Hz, 1H), 6.99 (t, J=8.8 Hz, 1H), 6.89 (s, 1H), 3.05(s, 2H), 2.34(s, 3H). HR-MS(ESI): m/z Calcd. For C₁₉H₁₄BrClN₂O₂ 415.9927; found 414.9854, [M-H]⁻.

### Example 16:

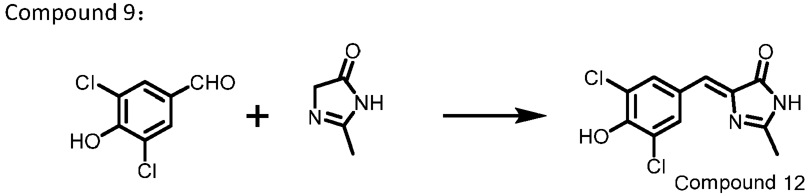

This compound was obtained by following the general procedure for Compound 5, (0.732 g, 93%) _{∘} ¹H NMR (400 MHz, CD₃OD) δ10.52 (s, 1H), 7.76 (d, J=8.5 Hz, 2H), 6.95 (s, 1H), 2.39(s, 3H). MS(ESI): m/z Calcd. For C₁₁H₈Cl₂N₂O₂ 270.0; found 271.0, [M+H]⁻.

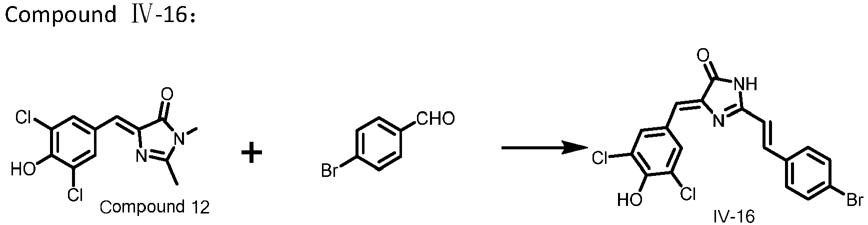

This compound was obtained by following the general procedure for Compound IV -1, (0.156 g, 18%) _{∘} ¹H NMR (400 MHz, CD₃OD) δ10.52 (s, 1H), 8.07 - 8.01 (m, 2H), 7.95 (d, J = 15.7 Hz, 1H),7.76 (d, J=8.5 Hz, 2H), 7.01 (d, J = 15.7 Hz, 1H),6.95 (s, 1H), 6.87 - 6.83 (m, 2H), 2.39(s, 3H).HR-MS(ESI): m/z Calcd. For C₁₈H₁₁BrCl₂N₂O₂ 435.9381; found 436.9459, [M+H]⁻.

### Example 17:

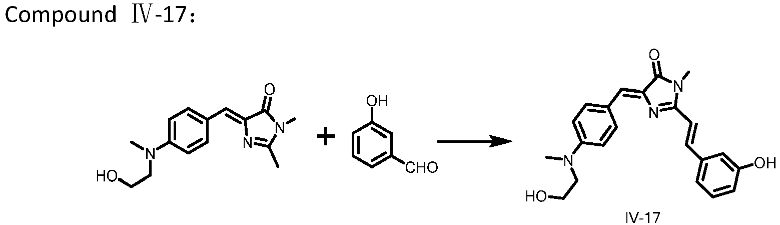

This compound was obtained by following the general procedure for CompoundIV-1, (0.286 g, 28%) _{∘} ¹H-NMR(400 MHz, DMSO-d₆): δ 8.15 (d, J = 8.6 Hz, 2H), 7.82 (d, J = 15.8 Hz, 1H), 7.31 - 7.22 (m, 2H), 7.21 - 7.17 (m, 1H), 7.10 (d, J = 15.8 Hz, 1H), 6.94 (s, 1H), 6.88 - 6.73 (m, 3H), 3.59 (t, J = 5.8 Hz, 2H), 3.51 (t, J = 6.0 Hz, 2H), 3.26 (s, 3H), 3.05 (s, 3H). HR-MS (ESI): m/z Calcd. For C₂₂H₂₄N₃O₃ 378.1818; found 378.1819, [M+H]⁺.

### Example 18:

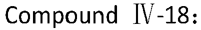

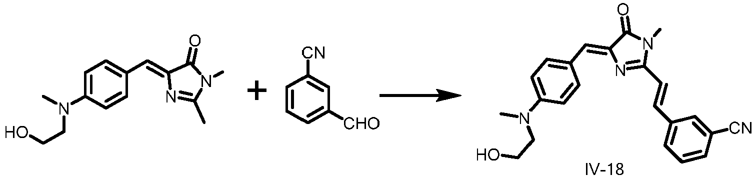

This compound was obtained by following the general procedure for CompoundIV -1, (0.486 g, 60%) _{∘} ¹H-NMR(400 MHz, DMSO- d₆): δ 8.43 (d, J = 1.8 Hz, 1H), 8.16 (t, J = 8.5 Hz, 3H), 7.93 (d, J = 15.9 Hz, 1H), 7.85 (dt, J = 7.8, 1.4 Hz, 1H), 7.66 (t, J = 7.8 Hz, 1H), 7.40 (d, J = 15.9 Hz, 1H), 6.98 (s, 1H), 6.86-6.71 (m, 2H), 3.60 (t, J = 5.9 Hz, 2H), 3.52 (t, J = 5.9 Hz, 2H), 3.28 (s, 3H), 3.06 (s, 3H). HR-MS(ESI): m/z Calcd. For C₂₃H₂₃N₄O₂ 387.1821; found 387.1822, [M+H]⁺.

### Example 19:

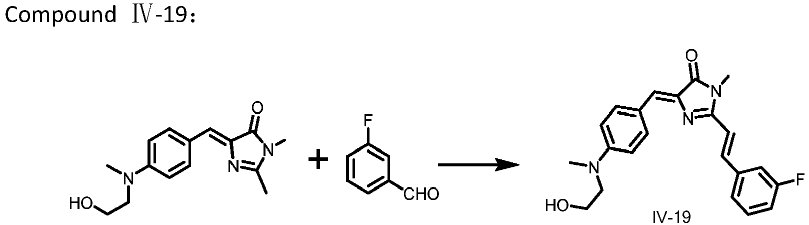

This compound was obtained by following the general procedure for CompoundIV-1, (0.286 g, 38%) _{∘} ¹H-NMR(400 MHz, DMSO-d₆): δ 8.17 (d, J = 8.6 Hz, 2H), 7.91 (d, J = 15.8 Hz, 1H), 7.79 (dt, J = 10.6, 2.1 Hz, 1H), 7.66 (d, J = 7.8 Hz, 1H), 7.50 (td, J = 8.0, 6.2 Hz, 1H), 7.34-7.20 (m, 2H), 6.97 (s, 1H), 6.84-6.73 (m, 2H), 3.59 (t, J = 5.9 Hz, 2H), 3.52 (t, J = 5.9 Hz, 2H), 3.27 (s, 3H), 3.06 (s, 3H). MS(ESI): m/z Calcd. For C₂₂H₂₃N₃O₃F 380.1774; found 380.1775, [M+H]⁺.

### Example 20:

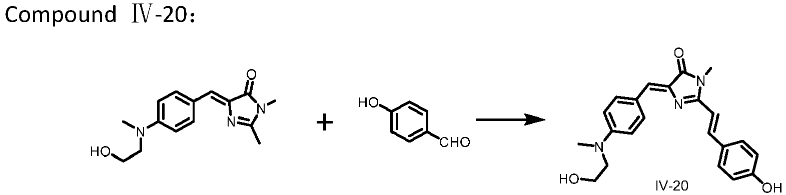

This compound was obtained by following the general procedure for CompoundIV-1, (0.222 g, 30%). ¹H NMR (400 MHz, DMSO-*d*₆): 8.14 (d, *J* = 8.5 Hz, 2H), 7.84 (d, *J* = 15.7 Hz, 1H), 7.68 (d, *J* = 8.4 Hz, 2H), 6.97 (d, *J* = 15.8 Hz, 1H), 6.88 (s, 1H), 6.84 (d, *J* = 8.5 Hz, 2H), 6.79 (d, *J* = 8.8 Hz, 2H), 3.59 (q, *J* = 5.4 Hz, 2H), 3.51 (t, *J* = 5.9 Hz, 2H), 3.24 (s, 3H), 3.05 (s, 3H). MS(ESI): m/z Calcd. For C₂₂H₂₄N₃O₃ 378.1818; found 378.1819, [M+H]⁺.

### Example 21:

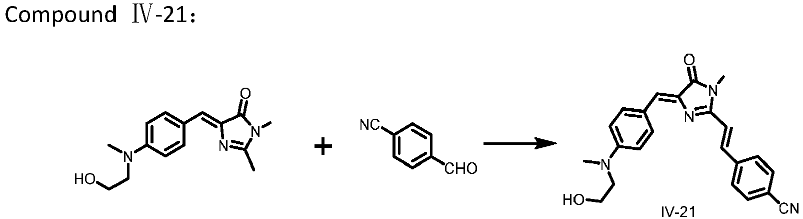

This compound was obtained by following the general procedure for CompoundIV-1, (0.352 g, 56%)_{∘} ¹H-NMR(400 MHz, DMSO-d₆): δ 8.18 (d, J = 8.5 Hz, 2H), 8.08-8.02 (m, 2H), 7.98-7.90 (m, 3H), 7.40 (d, J = 15.9 Hz, 1H), 7.00 (s, 1H), 6.86-6.72 (m, 2H), 3.60 (t, J = 5.9 Hz, 2H), 3.52 (t, J = 5.6 Hz, 2H), 3.28 (s, 3H), 3.06 (s, 3H). MS(ESI): m/z Calcd. For C₂₃H₂₃N₄O₂ 387.1821; found 387.1820, [M+H]⁺.

### Example 22:

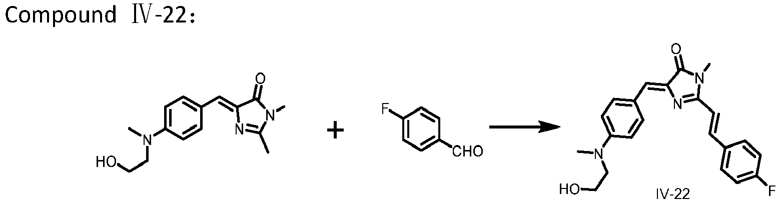

This compound was obtained by following the general procedure for CompoundIV -1, (0.252 g, 32%)_{∘} ¹H-NMR(400 MHz, DMSO-d₆): δ 8.16 (d, *J* = 8.5 Hz, 2H), 7.93 (t, *J* = 4.4 Hz, 2H), 7.90 (d, *J* = 6.1 Hz, 1H), 7.31 (t, *J* = 8.9 Hz, 2H), 7.18 (d, *J* = 15.9 Hz, 1H), 6.95 (s, 1H), 6.83-6.72 (m, 2H), 3.59 (t, *J* = 5.9 Hz, 2H), 3.51 (t, *J* = 5.9 Hz, 2H), 3.27 (s, 3H), 3.05 (s, 3H). MS(ESI): m/z Calcd. For C₂₂H₂₃FN₃O₂ 380.1774; found 380.1775, [M+H]⁺.

### Example 23:

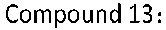

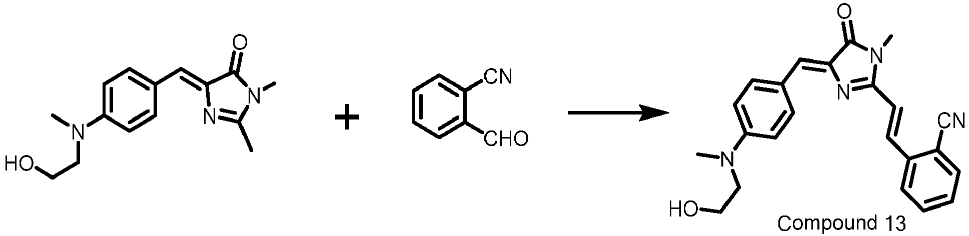

This compound was obtained by following the general procedure for CompoundIV -1, (0.252 g, 32%) _{∘} ¹H-NMR(400 MHz, DMSO-d₆): δ 8.43 (d, J = 1.8 Hz, 1H), 8.16 (t, J = 8.5 Hz, 3H), 7.93 (d, J = 15.9 Hz, 1H), 7.85 (dt, J = 7.8, 1.4 Hz, 1H), 7.66 (t, J = 7.8 Hz, 1H), 7.40 (d, J = 15.9 Hz, 1H), 6.98 (s, 1H), 6.86-6.71 (m, 2H), 3.60 (t, J = 5.9 Hz, 2H), 3.52 (t, J = 5.9 Hz, 2H), 3.28 (s, 3H), 3.06 (s, 3H). MS(ESI): m/z Calcd. For C₂₃H₂₃N₄O₂ 387.2; found 387.2, [M+H]⁺.

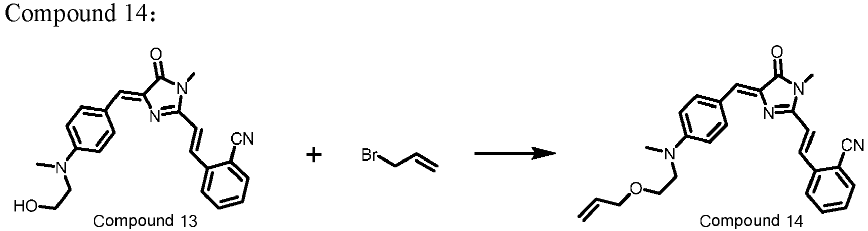

2 ml Allyl bromide was added to the mixture of compound 13 (0.774 g, 2.0 mmol), K₂CO₃ (0.276g, 2.0 mmol) in acetonitrile (100 ml) with constant stirring. This reaction mixture was heated to reflux. The progress of reaction was monitored on silica gel TLC. After completion of reaction, the reaction mixture was filtered then the solvent was removed under reduce pressure to give the crude product, then purified by silica gel column chromatography to afford a target compound 14. ¹H-NMR(400 MHz, DMSO-d₆): δ 8.43 (d, J = 1.8 Hz, 1H), 8.16 (t, J = 8.5 Hz, 3H), 7.93 (d, J = 15.9 Hz, 1H), 7.85 (dt, J = 7.8, 1.4 Hz, 1H), 7.66 (t, J = 7.8 Hz, 1H), 7.40 (d, J = 15.9 Hz, 1H), 6.98 (s, 1H), 6.86-6.71 (m, 2H), 3.81 (s,2H), 3.60 (t, J = 5.9 Hz, 2H), 3.52 (t, J = 5.9 Hz, 2H), 3.41 (s, 3H), 3.06 (s, 3H). MS(ESI): m/z Calcd. For C₂₆H₂₇N₄O₂ 427.2; found 427.2, [M+H]⁺.

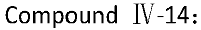

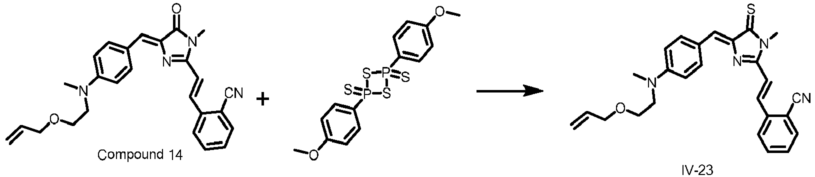

This compound was obtained by following the general procedure for CompoundIV-7, (0.152 g, 72%) _{∘} ¹H-NMR(400 MHz, DMSO-d₆): δ 8.43 (d, J = 1.8 Hz, 1H), 8.16 (t, J = 8.5 Hz, 3H), 7.93 (d, J = 15.9 Hz, 1H), 7.85 (dt, J = 7.8, 1.4 Hz, 1H), 7.66 (t, J = 7.8 Hz, 1H), 7.40 (d, J = 15.9 Hz, 1H), 6.98 (s, 1H), 6.86-6.71 (m, 2H), 3.81 (s,2H), 3.60 (t, J = 5.9 Hz, 2H), 3.52 (t, J = 5.9 Hz, 2H), 3.41 (s, 3H), 3.06 (s, 3H). MS(ESI): m/z Calcd. For C₂₆H₂₇N₄OS 443.1906; found 443.1905, [M+H]⁺.

### Example 24:

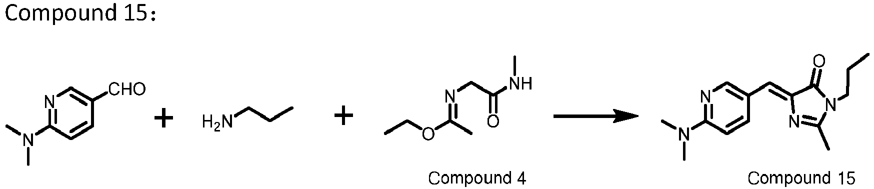

This compound was obtained by following the general procedure for CompoundIV-5, (0.692 g, 82%) _{∘} ¹H NMR (400 MHz, CD₃OD) δ =8.02 (d, J= 2.4 Hz, 1H), 7.44 (dd, J= 8.7 Hz, J= 2.4 Hz, 1H), 7.09 (s, 1H), 6.51 (d, J= 8.7 Hz, 1H), 3.56 (t, J HH = 7.6 Hz, 2H), 3.08 (s, 6H), 1.66 (m, 2H), 2.38 (s, 3H), 0.95 (t, J = 7.6 Hz, 3H). MS(ESI): m/z Calcd. For C₁₅H₂₁N₄O 273.2; found 273.2, [M+H]⁺.

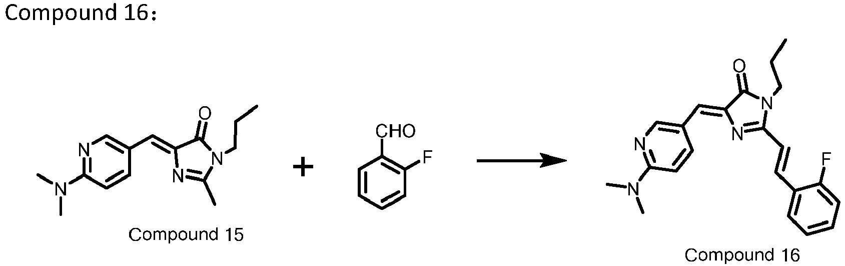

This compound was obtained by following the general procedure for Compound IV -1, (0.452 g, 34%) _{∘} ¹H NMR (400 MHz, CD₃OD) δ =8.02 (d, J= 2.4 Hz, 1H), 7.95 (m, 2H), 7.68-7.50 (m, 1H), 7.44 (dd, J= 8.7 Hz, J= 2.4 Hz, 1H), 7.34-7.06 (m, 2H), 7.09 (s, 1H), 7.00 (d, J = 15.7 Hz, 1H), 6.51 (d, J= 8.7 Hz, 1H), 3.56 (t, J HH = 7.6 Hz, 2H), 3.08 (s, 6H), 1.66 (m, 2H), 2.38 (s, 3H), 0.95 (t, J = 7.6 Hz, 3H). MS(ESI): m/z Calcd. For C₂₂H₂₄FN₄O 379.2; found 379.2, [M+H]⁺.

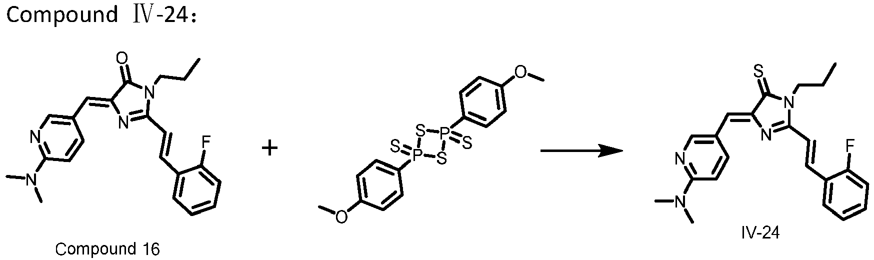

This compound was obtained by following the general procedure for Compound IV -7, (0.152 g, 72%) _{∘} ¹H NMR (400 MHz, CD₃OD) δ =8.02 (d, J= 2.4 Hz, 1H), 7.95 (m, 2H), 7.68-7.50 (m, 1H), 7.44 (dd, J= 8.7 Hz, J= 2.4 Hz, 1H), 7.34-7.06 (m, 2H), 7.09 (s, 1H), 7.00 (d, J = 15.7 Hz, 1H), 6.51 (d, J= 8.7 Hz, 1H), 3.56 (t, J HH = 7.6 Hz, 2H), 3.08 (s, 6H), 1.66 (m, 2H), 2.38 (s, 3H), 0.95 (t, J = 7.6 Hz, 3H). HR-MS(ESI): m/z Calcd. For C₂₂H₂₄FN₄S 395.1706; found 395.1705,
[M+H]⁺.

### Example 25:

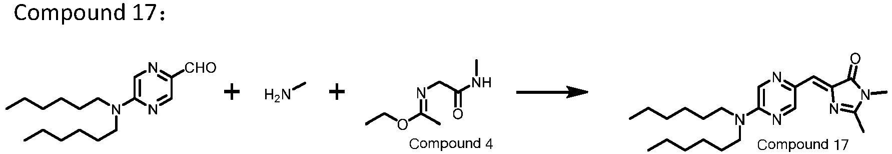

This compound was obtained by following the general procedure for CompoundIV-5, (0.892 g, 80%) _{∘} ¹H NMR (400 MHz, CD₃OD) δ8.41 (d, J = 1.5 Hz, 1 H), 7.97 (d, J = 1.5 Hz, 1 H), 7.31(s, 1H), 5.86 (s, 1 H), 3.46 (t, J = 6.6 Hz, 4 H), 3.15(s, 3H), 2.32(s, 3H), 1.61 (m, 4 H),1.32 (m, 12 H), 0.89 (t, 6 H). MS(ESI): m/z Calcd. For C₂₂H₃₆N₅O 386.3; found 386.3, [M+H]⁺.

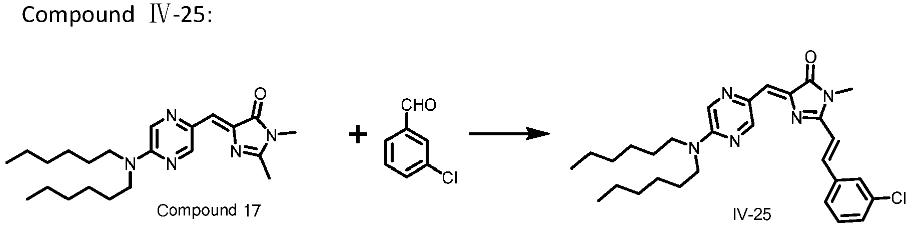

This compound was obtained by following the general procedure for Compound IV -1, (0.452 g, 34%) _{∘} ¹H NMR (400 MHz, CD₃OD) δ8.41 (d, J = 1.5 Hz, 1 H), 7.97 (d, J = 1.5 Hz, 1 H), 7.85 (d, J = 15.7 Hz, 1H), 7.49(m, 1H), 7.40-7.22(m, 3H), 7.31(s, 1H), 7.01 (d, J = 15.7 Hz, 1H), 5.86 (s, 1 H), 3.46 (t, J = 6.6 Hz, 4 H), 3.15(s, 3H), 2.32(s, 3H), 1.61 (m, 4 H),1.32 (m, 12 H), 0.89 (t, 6 H). MS(ESI): m/z Calcd. For C₂₉H₃₉ClN₅O 508.2843; found 508.2842, [M+H]⁺.

### Example 26:

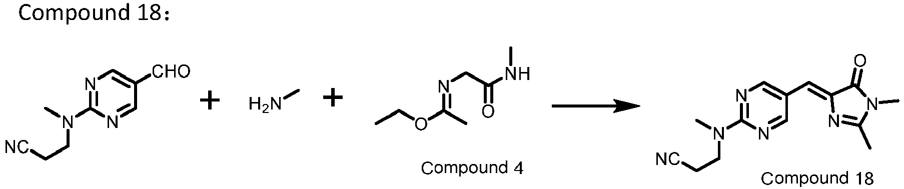

This compound was obtained by following the general procedure for CompoundIV-5, (0.812 g, 81%) _{∘} ¹H NMR (400 MHz, CDCl₃) δ 7.93(s, 2H), 7.31 (s, 1H), 4.24(t, J=6.8 Hz, 2H), 3.44 (s, 3H), 2.82(s, 2H), 2.43(s, 3H). MS(ESI): m/z Calcd. For C₁₄H₁₇N₆O 285.1; found 285.1, [M+H]⁺.

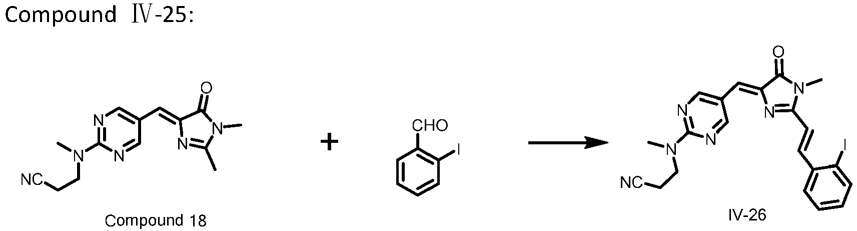

This compound was obtained by following the general procedure for Compound IV -1, (0.312 g, 26%) _{∘} ¹H NMR (400 MHz, CDCl₃) δ =8.02 (d, J = 15.7 Hz, 1H),7.93(m, 3H), 7.68-7.50 (m, 1H),7.31 (s, 1H), 7.24-7.06 (m, 2H), 7.01 (d, J = 15.7 Hz, 1H), 4.24(t, J=6.8 Hz, 2H), 3.44 (s, 3H), 2.82(s, 2H), 2.43(s, 3H). MS(ESI): m/z Calcd. For C₂₁H₂₀IN₆O 499.0743; found 499.0742, [M+H]⁺.

### Example 27:

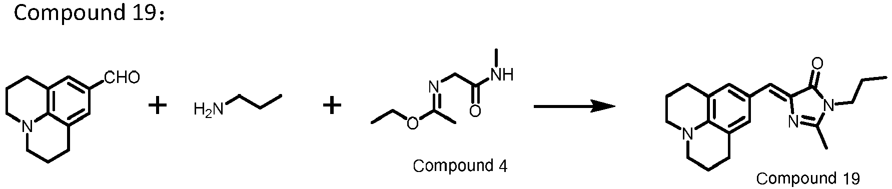

This compound was obtained by following the general procedure for CompoundIV-5, (0.932 g, 85%) _{∘} ¹H NMR (400 MHz, CDCl₃) δ=7.59 (s, 2 H), 6.71 (s, 1 H), 3.24 (t, *J*=5.6 Hz, 4 H), 3.06 (t, J=8.4 Hz, 2 H), 2.67 (t, J=6.2 Hz, 4 H), 2.29 (s, 3 H), 1.86-1.82 (m, 4 H) , 1.46 (m, 2 H), 1.21(t, J=8.4 Hz, 3H). MS(ESI): m/z Calcd. For C₂₀H₂₅N₃O 323.2; found 324.2, [M+H]⁺.

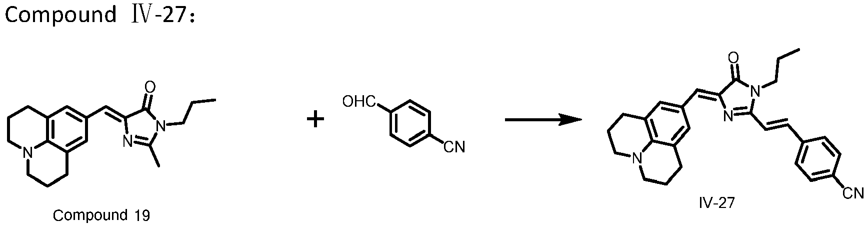

This compound was obtained by following the general procedure for CompoundIV-5, (0.932 g, 85%) _{∘} ¹H NMR (400 MHz, CDCl₃) δ= 8.31(dd, J=8.3 Hz, J=2.1 Hz, 1H), 8.16 (s, 1H), 7.94(d, J=8.3 Hz, 1H), 7.95 (d, J = 15.7 Hz, 1H), 7.59 (s, 2 H), 7.01 (d, J = 15.7 Hz, 1H), 6.71 (s, 1 H), 3.24 (t, *J*=5.6 Hz, 4 H), 3.06 (t, J=8.4 Hz, 2 H), 2.67 (t, *J*=6.2 Hz, 4 H), 2.29 (s, 3 H), 1.86-1.82 (m, 4 H), 1.46 (m, 2 H), 1.21(s, J=8.4 Hz, 3H). MS(ESI): m/z Calcd. For C₂₈H₂₈N₄O₂ 436.2263; found 437.2340, [M+H]⁺.

### Example 28:

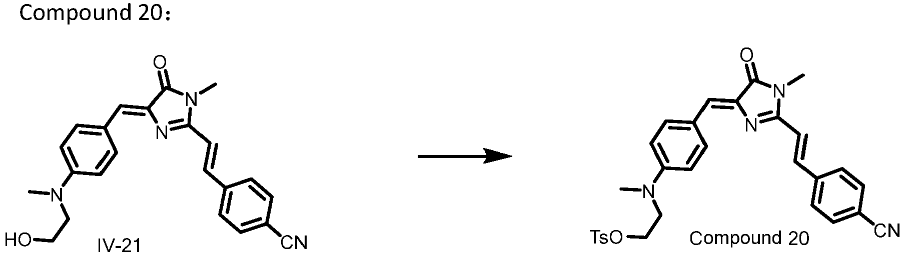

To a stirred solution of compound IV-21 (0.792 g, 2.0 mmol), p-toluenesulfonyl chloride (0.476 g, 2.5 mmol) in 100 mL dry DCM, TEA (0.303 g, 3.0 mmol) was added at rt under Ar atomophere. The progress of reaction was monitored on silica gel TLC. After completion of reaction, the reaction was poured into 200 mL water, and extracted with DCM three times. The organic layer was dried over anhydrous MgSO₄, filtered, and concentrated under reduced pressure to give the crude product, then purified by silica gel column chromatography to afford a target compound 27 (0.822 g, 76%).¹H-NMR(400 MHz,DMSO-d₆): δ 8.18 (d, J = 8.5 Hz, 2H), 8.08-8.02 (m, 2H), 7.98-7.90 (m, 3H), 7.40 (d, J = 15.9 Hz, 1H), 7.18 (d, *J*=8.2 Hz, 2H), 7.00 (s, 1H), 6.86-6.72 (m, 2H), 6.47 (d, *J*=8.2 Hz, 2H), 4.06 (t, *J*=6.1 Hz, 2H), 3.49 (t, *J*=6.1 Hz, 2H),3.28 (s, 3H), 2.77 (s, 3H), 2.31 (s, 3H). MS(ESI): m/z Calcd. For C₃₀H₂₉N₄O₄S 541.2; found 541.2, [M+H]⁺.

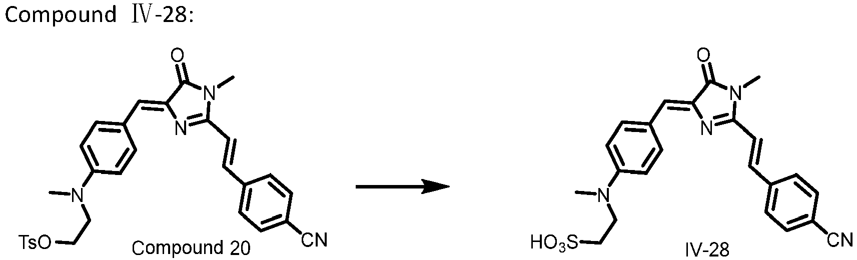

To a stirred solution of compound 20 (0.541 g, 1.0 mmol) in 20 mL dry DMF, sodium sulfite (0.630 g, 5.0 mmol) was added, the mixture solution was heated to 50 °C and stirred for 24 hrs under Ar atomophere. The progress of reaction was monitored on silica gel TLC. After completion of reaction, the solvent was removed under reduced pressure to give the crude product, then purified by silica gel column chromatography to afford a target compound 27 (0.301 g, 60%). ¹H-NMR(400 MHz,DMSO-d₆): δ 8.18 (d, J = 8.5 Hz, 2H), 8.08-8.02 (m, 2H), 7.98-7.90 (m, 3H), 7.40 (d, J = 15.9 Hz, 1H), 7.00 (s, 1H), 6.86-6.72 (m, 2H), 3.85 (m, 4H), 3.60 (t, J = 5.9 Hz, 2H), 3.52 (t, J = 5.6 Hz, 2H), 3.28 (s, 3H), 3.16 (m, 4H), 2.77 (s, 3H). MS(ESI): m/z Calcd. For C₂₃H₂₁N₄O₄S 499.1289; found 499.1288, [M-H]⁻.

### Example 29:

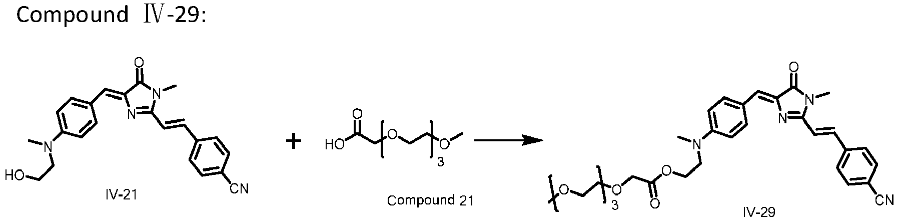

To a stirred solution of compound IV-21 (0.386 g, 1.0 mmol), compound 31 (0.265 g, 1.2 mmol), EDCI (0.382 g, 2.0 mmol) in 30 mL dry DMF, DMAP (0.183 g, 1.5 mmol) was added. The mixture solution was stirred at rt under Ar atomophere. The progress of reaction was monitored on silica gel TLC. After completion of reaction, the solvent was removed under reduced pressure to give the crude product, then purified by silica gel column chromatography to afford a target compound IV-29 (0.490 g, 83%). ¹H-NMR(400 MHz,DMSO-d₆): δ 8.18 (d, J = 8.5 Hz, 2H), 8.08-8.02 (m, 2H), 7.98-7.90 (m, 3H), 7.40 (d, J = 15.9 Hz, 1H), 7.00 (s, 1H), 6.86-6.72 (m, 2H), 4.17 (s, 2 H), 3.75 (s, 3 H), 3.6-3.7 (m, 10 H), 3.57 (m, 2H), 3.52 (t, J = 5.6 Hz, 2H), 3.38 (s, 3 H),3.28 (s, 3H), 3.06 (s, 3H). MS(ESI): m/z Calcd. For C₃₂H₃₉N₄O₇S 591.2819; found 591.2820, [M+H]⁺.

### Example 30:

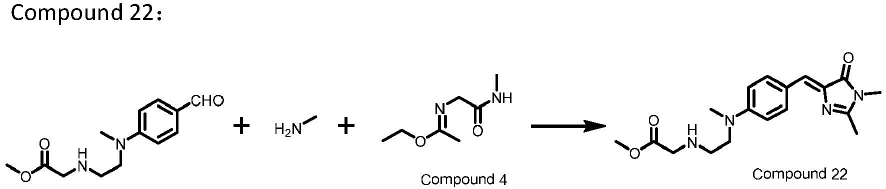

This compound was obtained by following the general procedure for CompoundIV-5, (0.612 g, 87%) _{∘} ¹H NMR (400 MHz, CDCl₃) δ 8.15 (d, J=9.0 Hz, 2 H), 8.14 (d, J=9.0 Hz, 2 H), 7.21 (s, 1H), 4.23(s, 2 H), 4.11 (s, 3 H), 3.38 (t, J = 6.4 Hz, 2 H), 3.01(s, 3H), 2.92 (t, J = 6.4 Hz, 2 H), 2.41 (s, 3H). MS(ESI): m/z Calcd. For C₁₈H₂₅N₄O₃ 345.2; found 345.2, [M+H]⁺.

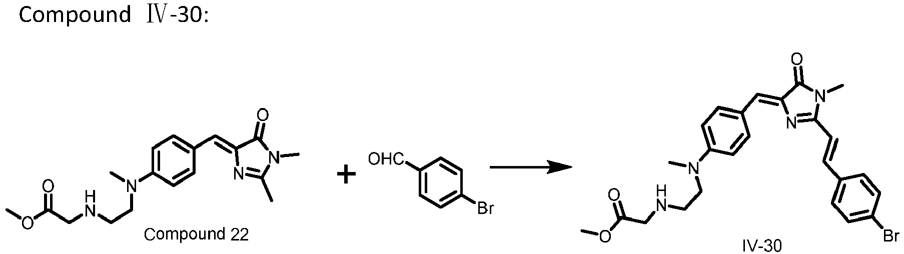

This compound was obtained by following the general procedure for CompoundIV-1, (0.422 g, 36%) _{∘} ¹H NMR (400 MHz, CDCl₃) δ 8.15 (d, J=9.0 Hz, 2 H), 8.14 (d, J=9.0 Hz, 2 H), 7.95 (d, J = 16.0 Hz, 1H), 7.82(d, J=8.4 Hz, 2H), 7.32 (d, J=8.4 Hz, 2H), 7.21 (s, 1H), 7.01 (d, J = 16.0 Hz, 1H), 4.23(s, 2 H), 4.11 (s, 3 H), 3.38 (t, J = 6.4 Hz, 2 H), 3.01(s, 3H), 2.92 (t, J = 6.4 Hz, 2 H).MS(ESI): m/z Calcd. For C₂₅H₂₈BrN₄O₃ 511.1345; found 511.1344, [M+H]⁺.

### Example 31:

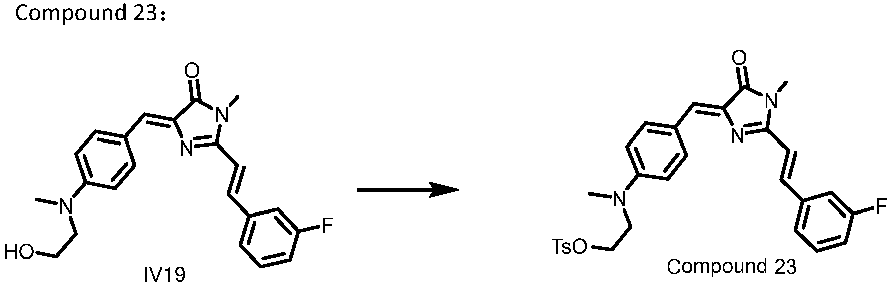

This compound was obtained by following the general procedure for CompoundIV-23, (0.912 g, 89%) _{∘} ¹H-NMR(400 MHz, DMSO-d₆): δ 8.17 (d, J = 8.6 Hz, 2H), 7.91 (d, J = 15.8 Hz, 1H), 7.79 (dt, J = 10.6, 2.1 Hz, 1H), 7.66 (d, J = 7.8 Hz, 1H), 7.50 (td, J = 8.0, 6.2 Hz, 1H), 7.34-7.20 (m, 2H), 7.18 (d, *J*=8.2 Hz, 2H), 6.97 (s, 1H), 6.84-6.73 (m, 2H), 6.47 (d, J=8.2 Hz, 2H), 4.06 (t, *J*=6.1 Hz, 2H), 3.49 (t, *J*=6.1 Hz, 2H), 3.27 (s, 3H), 3.06 (s, 3H), 2.77 (s, 3H), 2.31 (s, 3H). MS(ESI): m/z Calcd. For C₂₉H₂₉FN₃O₄S 534.2; found 534.2, [M+H]⁺.

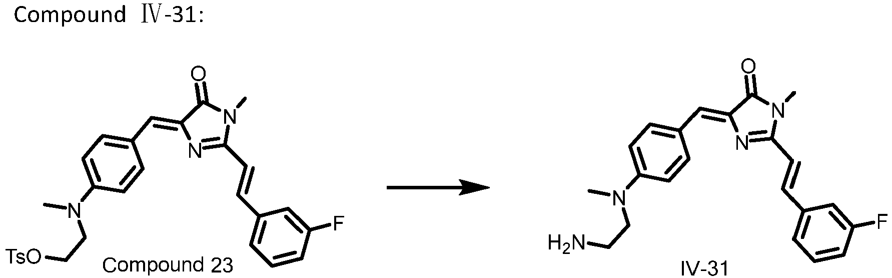

To a stirred solution of compound 23 (0.534 g, 1.0 mmol) in 35 mL dry DMF, NaN₃ (0.195 g, 3.0 mmol) was added carefully. The mixture solution heated to 50 °C over night under Ar atomophere. The solution was cooled down to rt and poured into 100 ml water and extracted with DCM for three times. The organic layer was dried over anhydrous MgSO₄, filtered, and concentrated under reduced pressure to give the crude product, which was used for next step without further purification.

To a stirred solution of crud production and Ph₃P (0.524 g, 2.0 mmol) in 30 mL THF , 2 mL water was added. The mixture solution was stirred at rt under Ar atomophere over night. After completion of reaction, the solvent was removed under reduced pressure to give the crude product, then purified by silica gel column chromatography to afford a target compound (0.301 g, 79%). ¹H-NMR(400 MHz, DMSO-d₆): δ 8.17 (d, J = 8.6 Hz, 2H), 7.91 (d, J = 15.8 Hz, 1H), 7.79 (dt, J = 10.6, 2.1 Hz, 1H), 7.66 (d, J = 7.8 Hz, 1H), 7.50 (td, J = 8.0, 6.2 Hz, 1H), 7.34-7.20 (m, 2H), 6.97 (s, 1H), 6.84-6.73 (m, 2H), 3.38 (t, J = 6.4 Hz, 2 H), 2.92 (t, J = 6.4 Hz, 2 H), 3.27 (s, 3H), 3.06 (s, 3H). MS(ESI): m/z Calcd. For C₂₂H₂₄FN₄O 379.1934; found 379.1935, [M+H]⁺.

### Example 32:

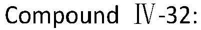

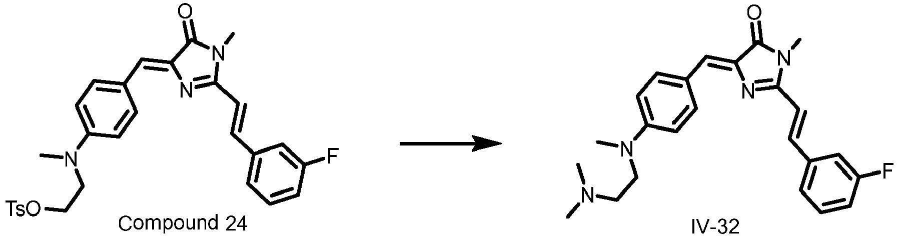

To a stirred solution of compound 24 (0.534 g, 1.0 mmol) in 50 mL ethanol, Dimethylamine aqueous solution, the complet mixture was heated to reflux under Ar atomophere. The progress of reaction was monitored on silica gel TLC. After completion of reaction, the reaction cooled down to rt and the solvent was removed under reduced pressure to give the crude product, then purified by silica gel column chromatography to afford a target compound (0.276 g, 68%). ¹H-NMR(400 MHz, DMSO-d₆): δ 8.17 (d, J = 8.6 Hz, 2H), 7.91 (d, J = 15.8 Hz, 1H), 7.79 (dt, J = 10.6, 2.1 Hz, 1H), 7.66 (d, J = 7.8 Hz, 1H), 7.50 (td, J = 8.0, 6.2 Hz, 1H), 7.34-7.20 (m, 2H), 6.97 (s, 1H), 6.84-6.73 (m, 2H), 3.47 (t, J = 7.6 Hz, 2H), 2.96 (s, 3H), 2.49 (t, J = 7.6 Hz, 2H), 2.31 (s, 6H). MS(ESI): m/z Calcd. For C₂₄H₂₈FN₄O 407.2247; found 407.2246, [M+H]⁺.

### Example 33:

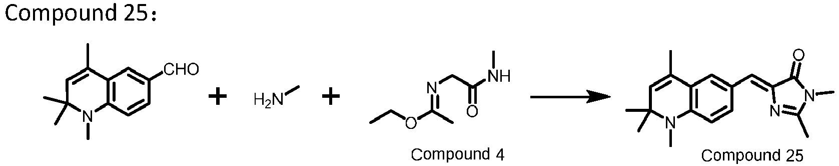

This compound was obtained by following the general procedure for CompoundIV-5, (0.842 g, 89%) _{∘} ¹H NMR (400 MHz, CDCl₃) δ=7.97 (d, J=8.6 Hz, 1 H), 7.88 (s, 1 H), 6.85 (s, 1 H), 6.56 (d, J=8.6 Hz, 1 H), 5.40 (s, 1 H), 3.07 (s, 3 H), 2.84 (s, 3 H), 2.31 (s, 3 H), 1.94 (s, 3 H), 1.32 (s, 6 H). MS(ESI): m/z Calcd. For C₁₉H₂₄N₃O 310.2; found 310.2, [M+H]⁺.

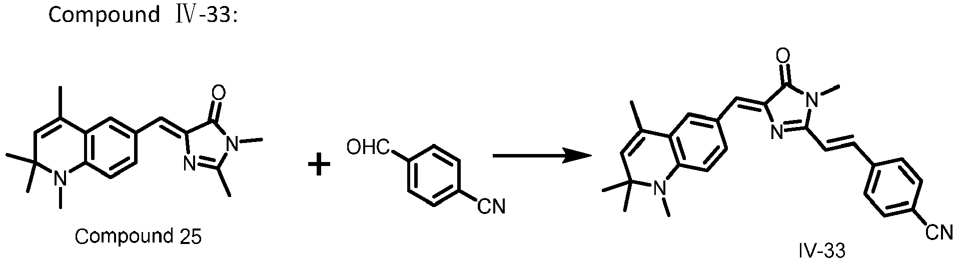

This compound was obtained by following the general procedure for Compound IV -1, (0.222 g, 21%) _{∘} ¹H NMR (400 MHz, CDCl₃) δ=7.97 (d, J=8.6 Hz, 1 H), 7.90 (d, J = 16.0 Hz, 1H), 7.88 (s, 1 H), 7.87 (d, J = 4.0 Hz, 2H ), 7.43 (d, J = 4.0 Hz, 2H), 7.01 (d, J = 16.0 Hz, 1H), 6.85 (s, 1H), 6.56 (d, J=8.6 Hz, 1H), 5.40 (s, 1 H), 3.07 (s, 3 H), 2.84 (s, 3 H), 1.94 (s, 3 H), 1.32 (s, 6 H).MS(ESI): m/z Calcd. For C₂₇H₂₆N₄O 422.2107; found 423.2186, [M+H]⁺.

### Example 34:

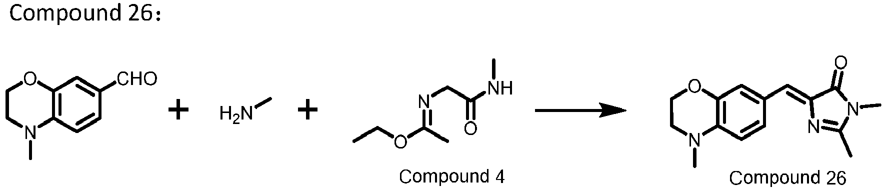

This compound was obtained by following the general procedure for CompoundIV-5, (0.732 g, 81%) _{∘} ¹H NMR (400 MHz, CDCl₃) δ=7.40 (dd, J=8.32, 1.93 Hz, 1H), 7.29 (d, J=1.89 Hz, 1 H),7.15(s, 1 H), 6.68 (d, J=8.35 Hz, 1 H), 4.23 - 4.31 (m, 2 H), 3.40 - 3.49 (m, 2 H),3.21(s, 3 H), 3.03 (s, 3 H), 2.42(s, 3 H). MS(ESI): m/z Calcd. For C₁₅H₁₈N₃O₂ 227.1; found 227.1, [M+H]⁺.

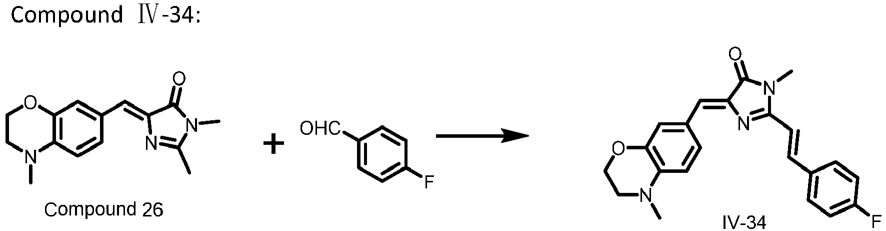

This compound was obtained by following the general procedure for CompoundIV-1, (0.222 g, 21%) _{∘} ¹H NMR (400 MHz, CDCl₃) δ=7.95 (d, J = 16.0 Hz, 1H), 7.87 (d, J = 4.0 Hz, 2H ), 7.43 (d, J = 4.0 Hz, 2H),7.40 (dd, J=8.32, 1.93 Hz, 1 H), 7.29 (d, J=1.89 Hz, 1 H),7.15(s, 1 H), 7.01 (d, J = 16.0 Hz, 1H), 6.68 (d, J=8.35 Hz, 1 H), 4.23 - 4.31 (m, 2 H), 3.40 - 3.49 (m, 2 H), 3.03 (s, 3 H), 2.42(s, 3 H).MS(ESI): m/z Calcd. For C₂₂H₂₀FN₃O₂ 377.1540; found 378.1681, [M+H]⁺.

### Example 35:

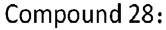

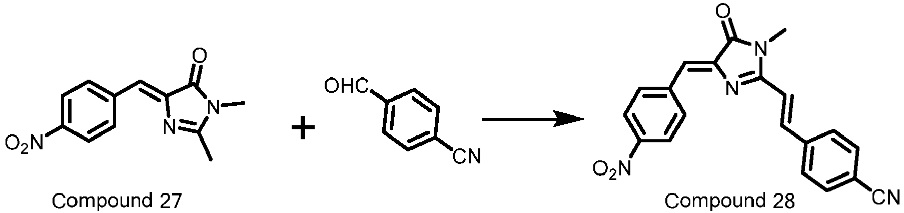

This compound was obtained by following the general procedure for Compound IV -1, (0.732 g, 39%) _{∘} ¹H NMR (400 MHz, CDCl₃) δ 8.19 (d, J = 8.5 Hz, 2H), 8.07 (m, 4H), 8.04 (s, 1H), 7.94 (d, J = 8.3 Hz, 2H), 7.44 (d, J = 15.9 Hz, 1H), 7.03 (s, 1H), 3.29 (s, 3H).. MS(ESI): m/z Calcd. For C₂₀H₁₅N₄O₃ 359.1; found 359.1, [M+H]⁺.

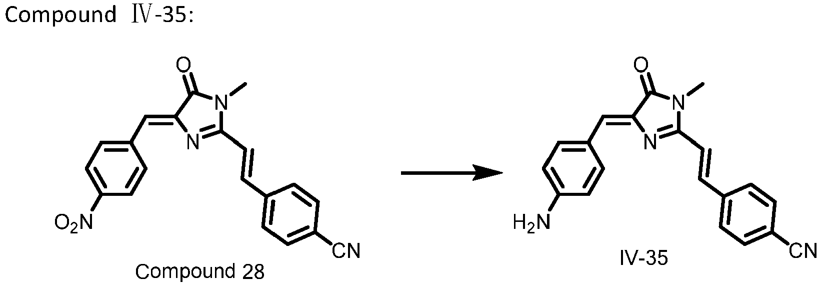

To a stirred solution of compound 28 (0.718 g, 2.0 mmol) in 100 mL ethyl acetate, Anhydrous stannous chloride (0.758 g, 4.0 mmol) was added. The complet mixture was heated to reflux under Ar atomophere. The progress of reaction was monitored on silica gel TLC. After completion of reaction, the reaction was poured into 150 mL water, and extracted with ethyl acetate for three times. The organic layer was dried over anhydrous MgSO₄, filtered, and concentrated under reduced pressure to give the crude product, then purified by silica gel column chromatography to afford a target compound (0.586 g, 89%).¹H NMR (400 MHz, CDCl₃) δ 8.19 (d, J = 8.5 Hz, 2H), 7.97 (m, 4H), 8.04 (s, 1H), 7.94 (d, J = 8.3 Hz, 2H), 7.44 (d, J = 15.9 Hz, 1H), 7.03 (s, 1H), 3.29 (s, 3H). MS(ESI): m/z Calcd. For C₂₀H₁₇N₄O 329.1402; found 329.1403, [M+H]⁺.

### Example 36:

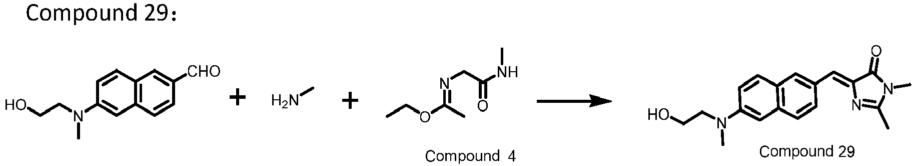

This compound was obtained by following the general procedure for CompoundIV-5, (0.756 g, 74%) _{∘} ¹H NMR (400 MHz, CDCl₃) δ 8.15 (1H, s), 7.81 (2H, m), 7.65 (1H, d, J = 9.0 Hz), 7.16 (1H, dd, J = 9.0, J = 3.0 Hz), 6.95 (s, 1H), 6.88 (1H, d, J = 3.0 Hz), 3.60 (t, J = 5.9 Hz, 2H), 3.52 (t, J = 5.9 Hz, 2H), 3.28 (s, 3H), 3.06 (s, 3H). MS(ESI): m/z Calcd. For C₁₉H₂₁N₃O₂ 323.2; found 324.2, [M+H]⁺.

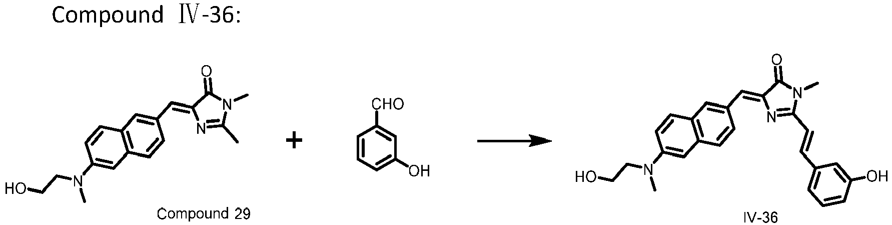

This compound was obtained by following the general procedure for CompoundIV-1, (0.272 g, 24%) _{∘} ¹H NMR (400 MHz, CDCl₃) δ 8.15 (1H, s), 7.81 (2H, m), 7.65 (1H, d, J = 9.0 Hz), 7.49(m, 1H), 7.40-7.22(m, 3H), 7.16 (1H, dd, J = 9.0, J = 3.0 Hz), 6.95 (s, 1H), 6.88 (1H, d, J = 3.0 Hz), 3.60 (t, J = 5.9 Hz, 2H), 3.52 (t, J = 5.9 Hz, 2H), 3.28 (s, 3H), 3.06 (s, 3H). MS(ESI): m/z Calcd. For C₂₆H₂₅N₃O₃ 427.1896; found 428.1974, [M+H]⁺.

### Example 37:

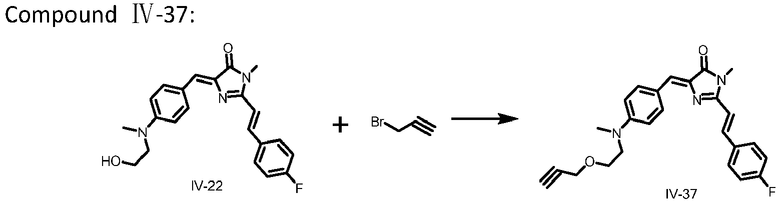

This compound was obtained by following the general procedure for CompoundIV-13, (0.322 g, 79%) _{∘} ¹H-NMR(400 MHz, DMSO-d₆): δ 8.16 (d, *J* = 8.5 Hz, 2H), 7.93 (t, *J* = 4.4 Hz, 2H), 7.90 (d, *J* = 6.1 Hz, 1H), 7.31 (t, *J* = 8.9 Hz, 2H), 7.18 (d, *J* = 15.9 Hz, 1H), 6.95 (s, 1H), 6.83-6.72 (m, 2H),4.21(s,2 H), 3.59 (t, *J* = 5.9 Hz, 2H), 3.51 (t, *J* = 5.9 Hz, 2H), 3.27 (s, 3H), 3.05 (s, 3H). MS(ESI): m/z Calcd. For C₂₅H₂₅FN₃O₂ 418.1931; found 418.1932, [M+H]⁺.

### Example 38:

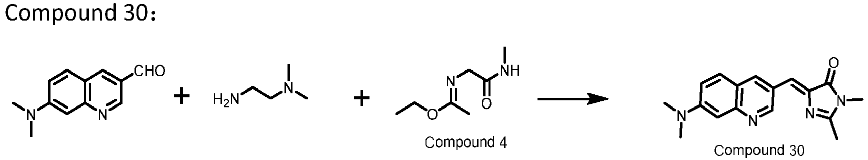

This compound was obtained by following the general procedure for CompoundIV-5, (0.816 g, 89%) _{∘} ¹H NMR (400 MHz, CDCl₃) δ 9.02 (d, 1 H, J = 2.1 Hz), 8.59 (d, 1 H, J = 2.1 Hz), 7.93 (d, 1 H, J = 9.1 Hz), 7.34 (dd, 1 H, J = 9.1, 2.5 Hz), 7.21(s, 1 H), 7.00 (d, 1 H, J = 2.5 Hz), 3.11 (s, 6 H), 3.06 (s, 3H). MS(ESI): m/z Calcd. For C₁₇H₁₈N₄O 294.1; found 295.1, [M+H]⁺.

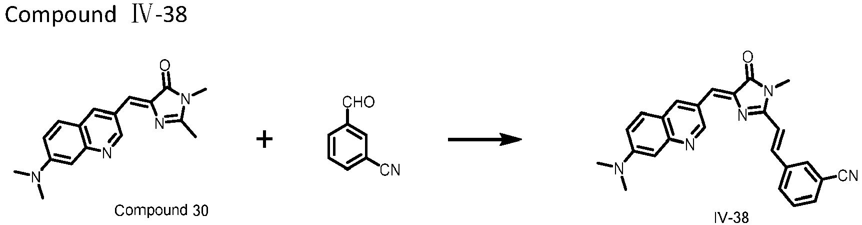

This compound was obtained by following the general procedure for CompoundIV-1, (0.272 g, 24%) _{∘} ¹H NMR (400 MHz, CDCl₃) δ =9.02 (d, 1 H, J = 2.1 Hz), 8.59 (d, 1 H, J = 2.1 Hz), 7.95 (d, J = 16.0 Hz, 1H), 7.93 (d, 1 H, J = 9.1 Hz), 7.49(m, 1H), 7.40-7.22(m, 3H),7.34 (dd, 1 H, J = 9.1, 2.5 Hz), 7.21(s, 1 H), 7.15 (d, J = 16.0 Hz, 1H),7.00 (d, 1 H, J = 2.5 Hz), 3.11 (s, 6 H), 3.06 (s, 3H). MS(ESI): m/z Calcd. For C₂₅H₂₁N₅O 407.1821; found 408.1825, [M+H]⁺.

### Comparative example 1:

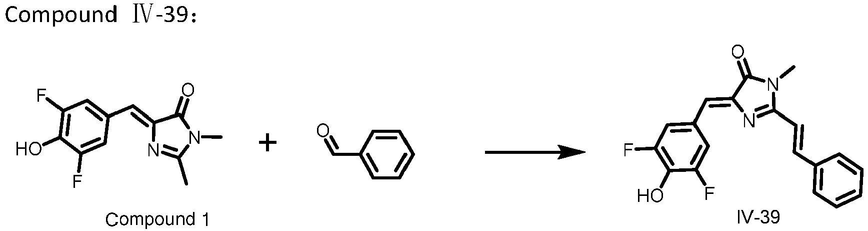

This compound was obtained by following the general procedure for CompoundIV-1, (0.275 g, 75%) _{∘} ¹H NMR (400 MHz, DMSO-*d*₆) δ 11.00 (s, 1H), 8.08-8.04 (m, 2H), 8.02 (d, *J* = 15.9 Hz, 1H), 7.92-7.87 (m, 2H), 7.49-7.45 (m, 2H), 7.26 (d, *J* = 15.9 Hz, 1H), 6.98 (s, 1H), 3.29 (s, 3H). MS(ESI): m/z Calcd. For C₁₉H₁₃F₂N₃O₂ 339.0951; found 339.0950, [M-H]⁻.

### Comparative example 2:

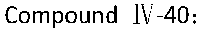

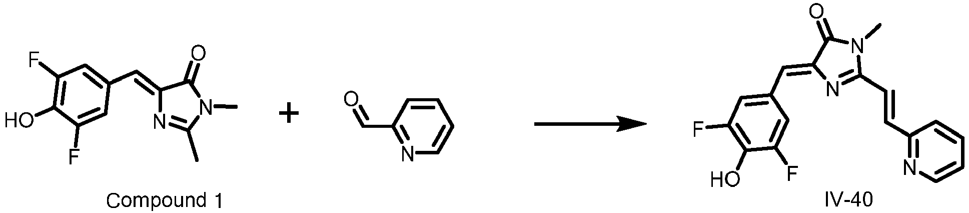

This compound was obtained by following the general procedure for Compound IV -1, (0.327 g, 55%) _{∘} ¹H NMR (400 MHz, DMSO-d₆) δ 9.70 (s, 1H), 8.05 (d, J = 8.9 Hz, 2H), 7.98 -7.89 (m, 1H), 7.30 (t, J = 6.2 Hz, 1H), 7.26 (d, J = 8.9 Hz, 1H), 7.15 (d, J = 15.8 Hz, 1H), 6.97 (s, 1H), 6.87 (d, J = 7.5 Hz, 1H), 3.27 (s, 3H). MS(ESI): m/z Calcd. For C₁₈H₁₂F₂N₃O₂ 340.0903; found 340.090, [M-H]⁻.

### Comparative example 3:

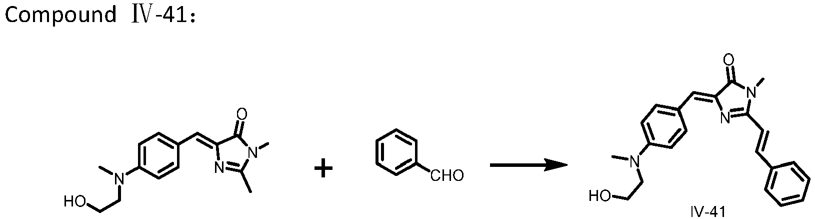

This compound was obtained by following the general procedure for CompoundIV-1, (0.342 g, 46%) _{∘} ¹H-NMR(400 MHz,DMSO-d₆): δ=8.21(d,2 H,J=8.8 Hz), 8.00(d,1 H,J=16 Hz), 7.85(d,2 H,J=8.0 Hz), 7.50-7.43 (m, 2H), 7.42 (d, *J* = 2.6 Hz, 1H), 7.24(s,1 H), 7. 0 1 (s, I H), 6.92(d,2 H,J=8.8 Hz), 3.85 (t,2H,J=5.6 Hz), 3.60(t,2 H,J=5.6 Hz),3.10(s, 3H). MS(ESI): m/z Calcd. For C₂₂H₂₄N₃O₂ 362.1869; found 362.1868, [M+H]⁺.

### Comparative example 4:

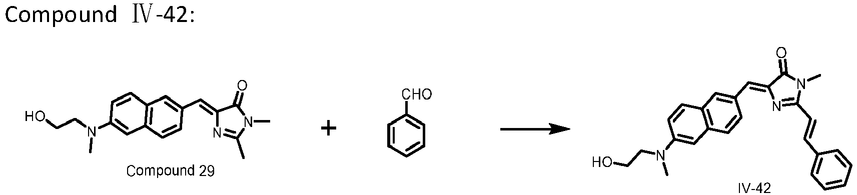

This compound was obtained by following the general procedure for Compound IV -1, (0.312 g, 43%) _{∘} ¹H NMR (400 MHz, CDCl₃) δ=8.15 (1H, s), 8.00(d,1 H,J=16 Hz), 7.85(d,2 H,J=8.0 Hz),7.81 (2H, m), 7.65 (1H, d, J = 9.0 Hz), 7.20(s, 1H), 7.50-7.43 (m, 3H), 7.42 (d,1 H,J=16 Hz),7.16 (1H, dd, J = 9.0, J = 3.0 Hz), 6.88 (1H, d, J = 3.0 Hz), 3.65(t, J=7.2 Hz, 2 H), 3.45(t, J=7.2 Hz, 2 H), 3.35(s, 3 H)3.03 (s, 3H).MS(ESI): m/z Calcd. For C₂₆H₂₆N₃O₂ 412.2025; found 412.2026, [M+H]⁺.

### Comparative example 5:

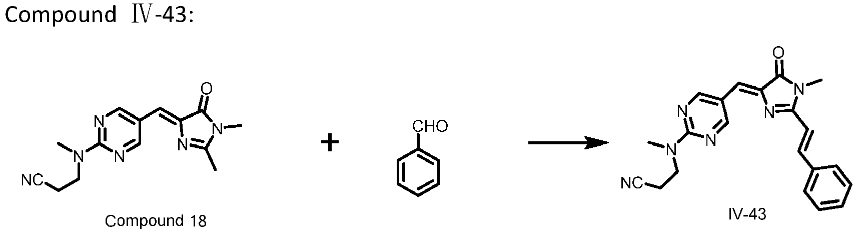

This compound was obtained by following the general procedure for Compound IV -1, (0.412 g, 46%) _{∘} ¹H-NMR(400 MHz,DMSO-d₆): δ=8.72 (s, 2H), 8.00(d,1 H,J=16 Hz), 7.50-7.43 (m, 2H), 7.42 (d, *J* = 2.6 Hz, 1H),7.24(s,1 H),7.01(s,1 H),6.92(d,2 H,J=8.8 Hz),3.75 (t, J= 6.8Hz, 2H), 3.60(t,2 H,J=6.8 Hz),3.05(s,3 H). MS(ESI): m/z Calcd. For C₂₁H₂₁IN₆O 373.1777; found 373.1778, [M+H]⁺.

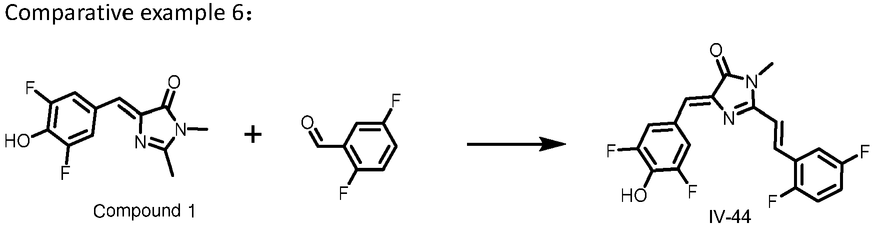

This compound was obtained by following the general procedure for CompoundIV-1, (0.192 g, 33%) _{∘} ¹H NMR (400 MHz, DMSO-*d*₆) δ 7.96-7.99 (m, 4H), 7.33-7.37 (m, 3H), 6.98 (s, 1H), 3.24(s, 3H). MS (ESI): m/z Calcd. For C₁₉H₁₂F₄N₂O₂ 376.0835; found 376.0830, [M-H]⁻.

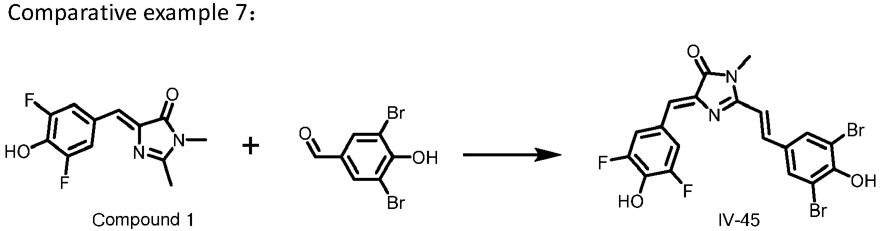

This compound was obtained by following the general procedure for CompoundIV-1, (0.192 g, 33%) _{∘} ¹H NMR (400 MHz, DMSO-*d*₆) δ 7.96-7.99 (m, 4H), 7.33-7.37 (m, 3H), 6.98 (s, 1H), 3.24(s, 3H). MS (ESI): m/z Calcd. For C₁₉H₁₂F₄N₂O₂ 376.0835; found 376.0830, [M-H]⁻.

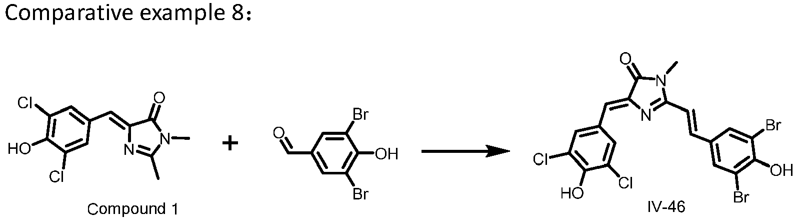

This compound was obtained by following the general procedure for CompoundIV-1, ¹H NMR (400 MHz, DMSO-d₆) δ ppm 10.70 ( s, 1H), 8.35 (s, 2H), 8.15 (s, 2H), 7.82 (d, J=15.8 Hz, 1H), 7.22 (d, J=15.9 Hz, 1H), 6.95 (s, 1H), 3.27 (s, 3H); HRMS (ESI) m/z: 546.8647 found (calcd for C₁₉H₁₂Br₂C1₂N₂O₃, [M+H]⁺ 546.8644).

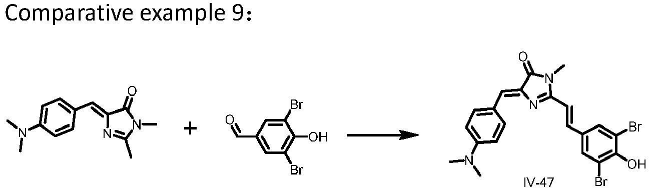

This compound was obtained by following the general procedure for CompoundIV-1, ¹H NMR (400 MHz, DMSO-d₆) δ ppm 10.38 (s, 1H), 8.18 (d, J=2.0 Hz, 2H), 8.12 (s, 2H), 7.81 (d, J=4.8 Hz, 1H), 7.18 (d, J=4.8 Hz, 1H), 6.93 (s, 1H), 6.78 (d, J=2.0 Hz, 2H), 3.25 (s, 3H), 3.04 (s, 6H); HRMS (ESI) m/z: 505.9855 found (calcd for C₂₁H₁₉Br₂N₃O₂, [M+H]⁺ 505.9896).

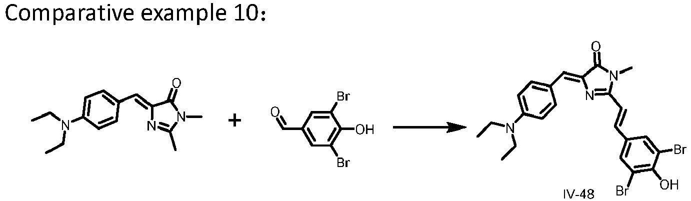

This compound was obtained by following the general procedure for CompoundIV-1, ¹H NMR (400 MHz, DMSO-d₆) δ ppm 10.38 ( s, 1H), 8.16 (d, J=2.0 Hz, 2H), 8.11 (s, 2H), 7.80 (d, J=4.8 Hz, 1H), 7.18 (d, J=4.8 Hz, 1H), 6.91 (s, 1H), 6.74 (d, J=2.0 Hz, 2H), 3.44 (d, J=4.8 Hz, 2H), 3.25 (s, 3H), 1.14 (t, J=4.8 Hz, 6H); HRMS (ESI) m/z: 534.0154 found (calcd for C₂₃H₂₃Br₂N₃O₂, [M+H]⁺ 534.0209).

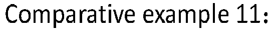

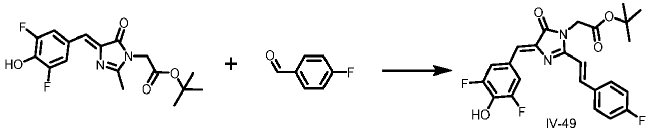

This compound was obtained by following the general procedure for CompoundIV-1, (0.156 g, 18%) _{∘} ¹H NMR (400 MHz, CD₃OD) δ10.52 (s, 1H), 8.07 - 8.01 (m, 2H), 7.95 (d, J = 15.7 Hz, 1H),7.76 (d, J=8.5 Hz, 2H), 7.01 (d, J = 15.7 Hz, 1H),6.95 (s, 1H), 6.87 - 6.83 (m, 2H), 4.12 (s, 3H), 3.62(s, 3H), 1.50(s, 9H).HR-MS(ESI): m/z Calcd. For C₂₄H₂₁F₃N₂O₄ 457.5381; found457.5380, [M-H]⁻.

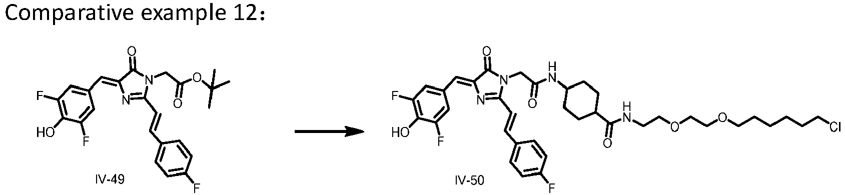

This compound was obtained by following the published procedure (JACS, 2018, 140, 7381-7384.)_{∘} ¹H NMR (400 MHz, CD₃OD) δ 8.17 (d, J=8.3 Hz, 2 H), 7.79 (d, J=15.6 Hz, 1 H), 7.55 (d, J=8.8 Hz, 2 H), 6.84 (s, 1 H), 6.80 (dd, J=9.9, 2.8 Hz, 2 H), 6.75 (dd, J=8.8, 5.6 Hz, 2 H), 6.66 (d, J=15.6 Hz, 1 H), 4.82(s, 2 H), 4.38 (s, 2 H), 3.59-3.43(m 8H), 3.41-3.35 (m, 2 H), 3.20-3.13 (m, 2 H), 3.04 (s, 6 H), 2.12-1.17 (m, 18 H). HR-MS(ESI): m/z Calcd. For C₃₇H₄₄ClF₃N₄O₆ 732.2901; found 733.2980, [M-H]⁻.

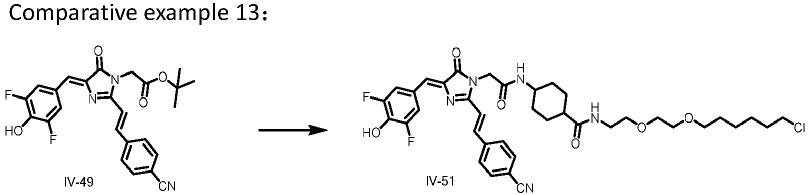

This compound was obtained by following the published procedure (JACS, 2018, 140, 7381-7384.)_{∘} ¹H NMR (400 MHz, CD₃OD) δ 8.17 (d, J=8.3 Hz, 2 H), 7.79 (d, J=15.6 Hz, 1 H), 7.55 (d, J=8.8 Hz, 2 H), 6.84 (s, 1 H), 6.80 (dd, J=9.9, 2.8 Hz, 2 H), 6.75 (dd, J=8.8, 5.6 Hz, 2 H), 6.66 (d, J=15.6 Hz, 1 H), 4.82(s, 2 H), 4.38 (s, 2 H), 3.59-3.43(m 8H), 3.41-3.35 (m, 2 H), 3.20-3.13 (m, 2 H), 3.04 (s, 6 H), 2.12-1.17 (m, 18 H). HR-MS(ESI): m/z Calcd. For C₃₈H₄₄ClF₃N₅O₆ 739.2948; found 740.3026, [M+H]⁻.

### Test Example 1:

Fluorescent dyes IV-1 to IV-38 (molecular rotors) prepared in Embodiments 1 to 38 were respectively dissolved in dimethyl sulfoxide to respectively prepare mother liquor with a concentration of 1×10⁻² M, wherein each mother liquor was respectively added to glycerin and methanol and blended, and thus respectively preparing a solution with a final concentration of 1×10⁻⁵ M; according to different fluorescent dyes, fluorescence emission spectra thereof were successively detected by means of the maximum excitation wavelength of each fluorescent dye under the same condition, and the results are shown in Table 1, indicating that the fluorescent dye of the present disclosure sensitively responds to viscosity variations.

**Table 1**

| Compounds | Maximum Excitation Wavelength (nm) | Glycerin/Methanol Fluorescence Intensity Ratio |
|---|---|---|
| IV-1 | 618 | 987 |
| IV-2 | 595 | 687 |
| IV-3 | 565 | 951 |
| IV-4 | 574 | 687 |
| IV-5 | 570 | 861 |
| IV-6 | 578 | 921 |
| IV-7 | 570 | 873 |
| IV-8 | 638 | 861 |
| IV-9 | 615 | 697 |
| IV-10 | 620 | 779 |
| IV-11 | 595 | 698 |
| IV-12 | 573 | 898 |
| IV-13 | 590 | 711 |
| IV-14 | 585 | 699 |
| IV-15 | 586 | 730 |
| IV-16 | 595 | 689 |
| IV-17 | 575 | 655 |
| IV-18 | 595 | 890 |
| IV-19 | 577 | 821 |
| IV-20 | 577 | 781 |
| IV-21 | 624 | 655 |
| IV-22 | 580 | 689 |
| IV-23 | 617 | 915 |
| IV-24 | 551 | 934 |
| IV-25 | 591 | 918 |
| IV-26 | 555 | 937 |
| IV-27 | 600 | 890 |
| IV-28 | 620 | 942 |
| IV-29 | 618 | 829 |
| IV-30 | 582 | 921 |
| IV-31 | 581 | 902 |
| IV-32 | 580 | 999 |
| IV-33 | 605 | 729 |
| IV-34 | 603 | 810 |
| IV-35 | 561 | 776 |
| IV-36 | 690 | 792 |
| IV-37 | 580 | 801 |
| IV-38 | 705 | 529 |

### Test Example 2:

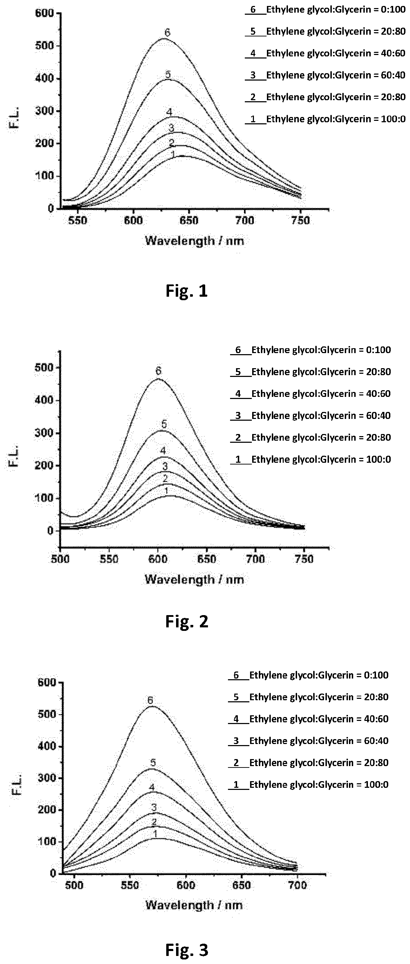

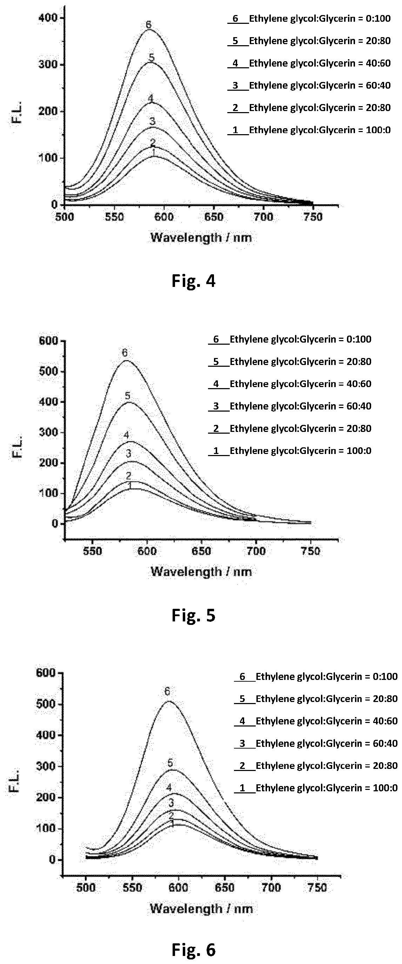

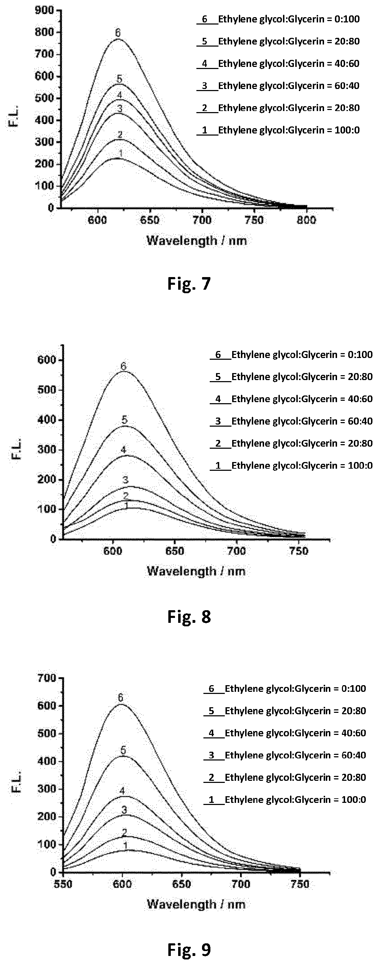

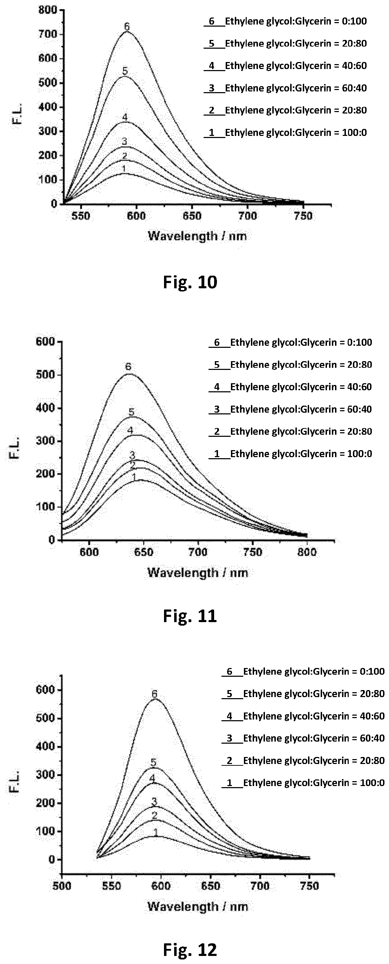

Molecular rotors IV-1, IV-2, IV-3, IV-4, IV-5, IV-6, IV-17, IV-18, IV-19, IV-20, IV-21, IV-22 were added to a diethanol-glycerol mixed solution to prepare a solution with a final concentration of 1×10⁻⁵ M, wherein the solution was excited at 480 nm, fluorescence emission spectra at different viscosity conditions are shown as Figs. 1, 2, 3, 4, 5, 6, 7, 8, 9, 10, 11, 12, and molecular rotors of the same concentration have gradually increasing fluorescence intensity at different viscosity conditions, indicating that the fluorescence intensity of molecular rotors increases following the increasing fluorescence of environmental viscosity, and proving that molecular rotors are sensitive to viscosity and are a kinds of molecular rotors.

### Test Example 3:

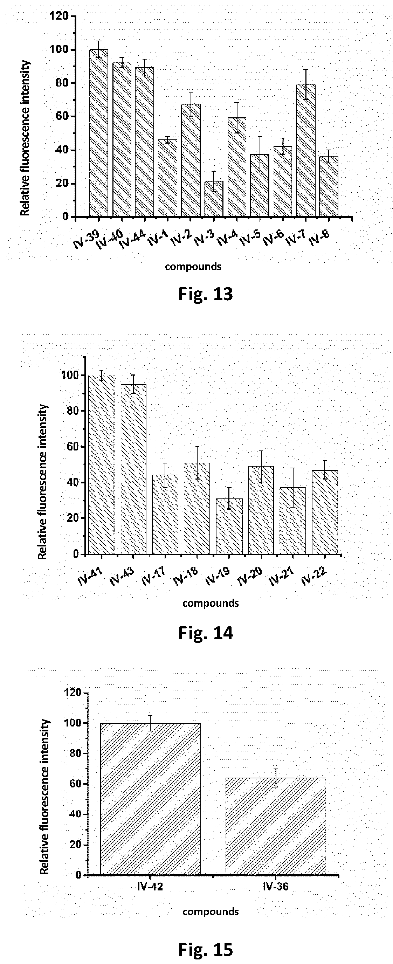

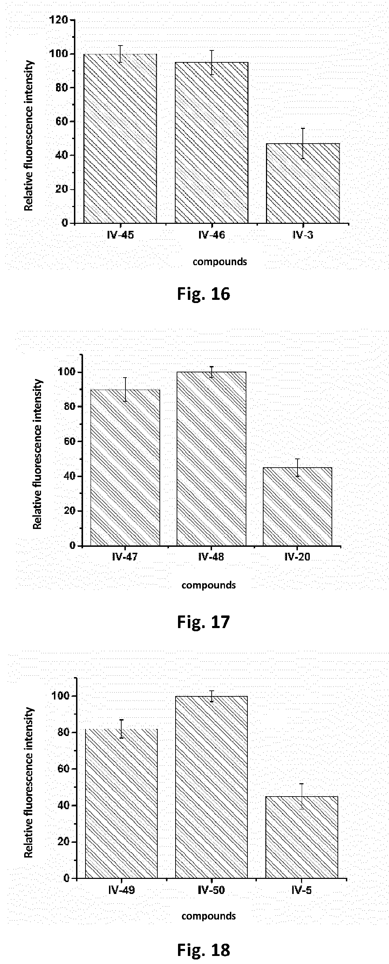

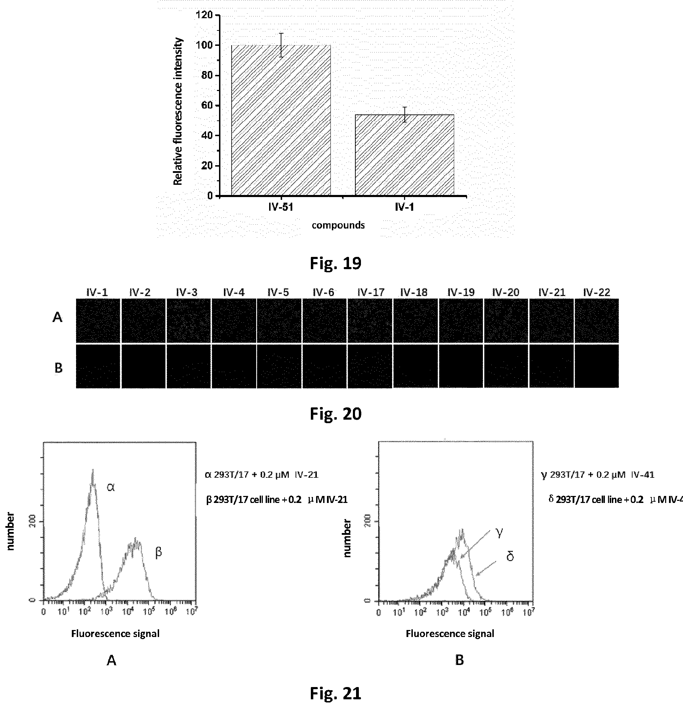

Molecular rotors (IV-39, IV-40, IV-44 and IV-1, IV-2, IV-3, IV-4, IV-5, IV-6, IV-7, IV-8; IV-41, IV-43 and IV-17, IV-18, IV-19, IV-20, IV-21, IV-22; IV-42 and IV-36; IV-45, IV-46 and IV-3; IV-47, IV-48 and IV-20; IV-49, IV-50 and IV-5; IV-51 and IV-1) were dissolved in DMSO to prepare mother liquor of 1×10⁻³ M, wherein the afore-mentioned mother liquor was respectively taken and added to a PBS solution to prepare a solution with a final concentration of 1×10⁻⁶ M, and was respectively excited by the maximum excitation wavelength of each compound, so that fluorescent intensities thereof in PBS were detected and each sample was normalized with the strongest fluorescence in each group as 100, as respectively shown in Fig. 13, Fig. 14, Fig. 15, Fig. 16, Fig. 17, Fig. 18 and Fig. 19. According to the results shown in Fig. 13, Fig. 14 and Fig. 15, relative to the molecular rotors with no substitution on the aromatic ring of the electron-withdrawing group, molecular rotors with hydroxyl radical, cyano group, fluorine atom, chlorine atom, bromine atom, iodine atom substitutions on the aromatic ring of the electron-withdrawing group in the present disclosure have lower background fluorescence; according to the results shown in Fig. 16 and Fig. 17, relative to the molecular rotors with multiple substitutions on the aromatic ring of the electron-withdrawing group, molecular rotors with single substitution have relatively lower background fluorescence, and it may be that the electron cloud distribution of the aromatic structure on the electron-withdrawing group is changed by the multiple substitutions, which causes an increase in the fluorescence background; according to the results shown in Fig. 18 and Fig. 19, the background fluorescence is even lower when simple alkyl substitution occurs at R₁, and it may be caused by relatively large volume of R₁ or weak interaction between heteroatoms (e.g. oxygen atoms, nitrogen atoms) on modified alkyl group and the aromatic ring on the electron-withdrawing group, as a result, non-radioactive energy dissipation motion such as double bond rotation is inhibited and the fluorescence background of the compound is thus increased.

### Test Example 4:

Compounds IV-1, IV-2, IV-3, IV-4, IV-5, IV-6, IV-17, IV-18, IV-19, IV-20, IV-21, IV-22 specifically bound to RNA aptamer (UUGCCAUGUGUAUGUGGGAGGAAGAUUGUAAACACGCCGGAAGAUUGUAAACACGCCGGAAGA UUGUAAACACGCCGGAAGAUUGUAAACACGCCGAAAGGCGGACACUUCCGGCGGACACUUCCGGC GGACACUUCCGGCGGACACUUCCUCCCACAUACUCUGAUGAUCCUUCGGGAUCAUUCAUGGCAA), and the compound fluorescence after binding was noticeably activated and emitted bright fluorescence when being excited by excitation light at a suitable wavelength, see Table 2 for the optical properties after binding; the compounds could also bind to this aptamer in cells, and cells expressing the RNA aptamer had bright fluorescence, as shown in Fig. 20A; and cells not expressing the RNA aptamer had no fluorescence, as shown in Fig. 20B, indicating that dyes of this series can be used for nucleic acid labeling.

**Table 2**

| Compounds | Ex/nm | Em/nm | ε (M⁻¹ cm⁻¹) | QY (-) |
|---|---|---|---|---|
| IV-1 | 510 | 618 | 20667 | 0.17 |
| IV-2 | 492 | 595 | 25000 | 0.36 |
| IV-3 | 490 | 565 | 26000 | 0.27 |
| IV-4 | 490 | 574 | 23333 | 0.37 |
| IV-5 | 492 | 570 | 23000 | 0.33 |
| IV-6 | 490 | 578 | 24000 | 0.37 |
| IV-17 | 524 | 582 | 32000 | 0.47 |
| IV-18 | 536 | 600 | 25000 | 0.43 |
| IV-19 | 522 | 590 | 30000 | 0.43 |
| IV-20 | 512 | 577 | 25500 | 0.36 |
| IV-21 | 534 | 624 | 26222 | 0.28 |
| IV-22 | 522 | 580 | 32000 | 0.46 |

Note: the fluorescence quantum yield was measured by the relative method with Rhodamine 6G as the standard (QY=0.94).

### Test Example 5:

Stable cell line (293T/17) of mRNA cytoskeletal protein, which is labeled by continuously-expressing RNA aptamer (AUGGAUGAUGAUAUCGCCGCGCUCGUCGUCGACAACGGCUCCGGCAUGUGCAAGGCCGGCUUCG CGGGCGACGAUGCCCCCCGGGCCGUCUUCCCCUCCAUCGUGGGGCGCCCCAGGCACCAGGGCGUGA UGGUGGGCAUGGGUCAGAAGGAUUCCUAUGUGGGCGACGAGGCCCAGAGCAAGAGAGGCAUCCU CACCCUGAAGUACCCCAUCGAGCACGGCAUCGUCACCAACUGGGACGACAUGGAGAAAAUCUGGCA CCACACCUUCUACAAUGAGCUGCGUGUGGCUCCCGAGGAGCACCCCGUGCUGCUGACCGAGGCCCC CCUGAACCCCAAGGCCAACCGCGAGAAGAUGACCCAGAUCAUGUUUGAGACCUUCAACACCCCAGC CAUGUACGUUGCUAUCCAGGCUGUGCUAUCCCUGUACGCCUCUGGCCGUACCACUGGCAUCGUGA UGGACUCCGGUGACGGGGUCACCCACACUGUGCCCAUCUACGAGGGGUAUGCCCUCCCCCAUGCC AUCCUGCGUCUGGACCUGGCUGGCCGGGACCUGACUGACUACCUCAUGAAGAUCCUCACCGAGCG CGGCUACAGCUUCACCACCACGGCCGAGCGGGAAAUCGUGCGUGACAUUAAGGAGAAGCUGUGCU ACGUCGCCCUGGACUUCGAGCAAGAGAUGGCCACGGCUGCUUCCAGCUCCUCCCUGGAGAAGAGC UACGAGCUGCCUGACGGCCAGGUCAUCACCAUUGGCAAUGAGCGGUUCCGCUGCCCUGAGGCACU CUUCCAGCCUUCCUUCCUGGGCAUGGAGUCCUGUGGCAUCCACGAAACUACCUUCAACUCCAUCA UGAAGUGUGACGUGGACAUCCGCAAAGACCUGUACGCCAACACAGUGCUGUCUGGCGGCACCACC AUGUACCCUGGCAUUGCCGACAGGAUGCAGAAGGAGAUCACUGCCCUGGCACCCAGCACAAUGAA GAUCAAGAUCAUUGCUCCUCCUGAGCGCAAGUACUCCGUGUGGAUCGGCGGCUCCAUCCUGGCCU CGCUGUCCACCUUCCAGCAGAUGUGGAUCAGCAAGCAGGAGUAUGACGAGUCCGGCCCCUCCAUC GUCCACCGCAAAUGCUUCUAGCACUCGCUAGAGCAUGGUUAAGCUUGGAAGAUUGUAAACACGCC GGAAGAUUGUAAACACGCCGGAAGAUUGUAAACACGCCGGAAGAUUGUAAACACGCCGAAAGGCG GACACUUCCGGCGGACACUUCCGGCGGACACUUCCGGCGGACACUUCC) and control cells (293T/17) grew under a conventional mammalian cell culture condition (37°C, 5% carbon dioxide, 100% relative humidity), and the cells were digested after a cell confluence of 90% and were centrifuged at 800 rpm; then the cells were re-suspended with PBS containing 0.2 µM of IV-21 and IV-41 molecules, and were incubated for 5 minutes before flow detection, see Fig. 21 for the detection results; IV-21 molecules could specifically label mRNA of ACTB in cell lines expressing target RNA, and there was no obvious background fluorescence (as shown in Fig. 21A), while the background fluorescence of IV-41 molecules was higher than that of IV-21, and it was unclear whether ACTB was expressed (see Fig. 21B).

## Claims

1. A fluorescent dye, which is shown as Formula (I), 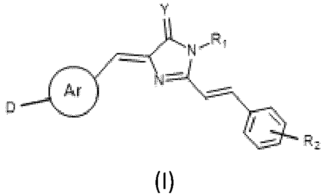 wherein:
Ar is arylene or heteroarylene, and optionally, hydrogen atoms in Ar are independently substituted by halogen atoms; D- is HO- or N(X₁)(X₂)-, and X₁ and X₂ are independently selected from hydrogen, alkyl or modified alkyl; X₁ and X₂ are optionally interconnected, and form an alicyclic heterocycle with N atoms; and X₁ and X₂ are optionally and independently form an alicyclic heterocycle with Ar;
Y is O or S;
R₁ is hydrogen or alkyl; and
R₂ is a halogen atom, -OH or -CN;
wherein:
the "alkyl" is C₁-C₁₀ straight or branched alkyl; optionally, the "alkyl" is C₁-C₆ straight or branched alkyl; optionally, the "alkyl" is C₁-C₄ straight or branched alkyl; optionally, the "alkyl" is selected from methyl, ethyl, n-propyl, isopropyl, n-butyl, isobutyl, tertiary butyl, sec-butyl, n-amyl, 1-methyl butyl, 2-methyl butyl, 3-methyl butyl, isoamyl, 1-ethyl propyl, neoamyl, n-hexyl, 1-methyl amyl, 2-methyl amyl, 3-methyl amyl, isohesyl, 1,1-dimethyl butyl, 2,2-dimethyl butyl, 3,3-dimethyl butyl, 1,2-dimethyl butyl, 1,3-dimethyl butyl, 2,3-dimethyl butyl, 2-ethyl butyl, n-heptyl, 2-methyl hexyl, 3-methyl hexyl, 2,2-dimethyl amyl, 3,3 dimethyl amyl, 2,3-dimethyl amyl, 2,4-dimethyl amyl, 3-ethyl amyl or 2,2,3-methyl butyl;
the "modified alkyl" is independently a group obtained by replacing any carbon atom in C₁-C₁₆ straight or branched alkyl with one or more groups selected from halogen atom, -OH, -CO-, -O-, -CN, -SO₃H-, primary amino group, secondary amino group and tertiary amino group, or the carbon-carbon single bond is optionally and independently replaced by a carbon-carbon double bond or a carbon-carbon triple bond;
the replacement of carbon atoms refers to that carbon atoms or the carbon atoms and hydrogen atoms thereon together are replaced by a corresponding group;
the "halogen atom" is independently F, Cl, Br or I;
the "alicyclic heterocycle" is a saturated or unsaturated 4- to 15-membered monocyclic or polycyclic alicyclic heterocycle containing one or more heteroatoms of N, O, S or Si on the ring, and the alicyclic heterocycle containing S heteroatom(s) on the ring include the ones that contain -S-, -SO- or -SO₂- on the ring; the alicyclic heterocycle is optionally substituted by a halogen atom, an alkyl, an aryl or a modified alkyl;
the "arylene" is independently a 5- to 13-membered (optionally 6- or 10-membered) monocyclic or dicyclic or fused dicyclic or fused polycyclic subaromatic group;
the "heteroarylene" is independently a 5- to 13-membered (optionally 6- or 10-membered) monocyclic or dicyclic or fused dicyclic or fused polycyclic sub-heteroaromatic group containing one or more heteroatoms selected from N, O, S or Si on the ring;
the "primary amino group" is R'NH₂ group;
the "secondary amino group" is R'NHR" group;
the "tertiary amino group" is R'NR"R‴ group;
each R', R", R‴ is independently a single bond, hydrogen, alkyl, or alkylene;
the "alkylene" is C₁-C₁₀ straight or branched alkylene; optionally, it is C₁-C₇ straight or branched alkylene; optionally, it is C₁-C₅ straight or branched alkylene.

2. The fluorescent dye according to claim 1, wherein the "modified alkylene" is a group containing one or more groups selected from -OH, -O-, -NH₂, ethylene glycol unit (-(CH₂CH₂O)ₙ-), -CN -O-CO-, -NH-CO-, -SO₂-O-, -SO-, Me₂N-, Et₂N-, -CH=CH-, -C≡CH, F, Cl, Br, I, and cyano group.

3. The fluorescent dye according to claim 1, wherein Ar is a structure selected from the following Formulae (II-1) to (II-7):

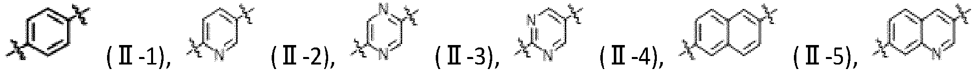

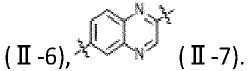

4. The fluorescent dye according to claim 1, wherein the compound represented by Formula (I) is selected from the compounds below:

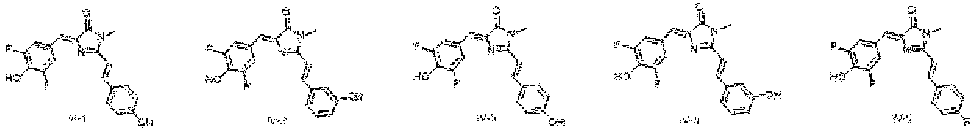

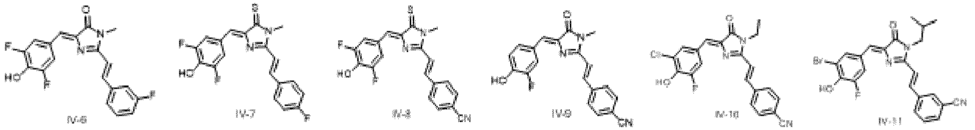

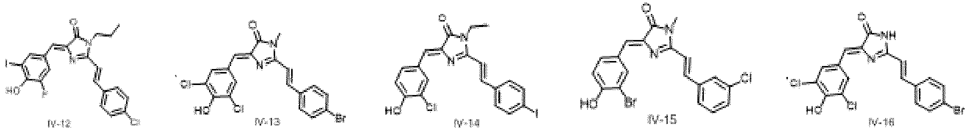

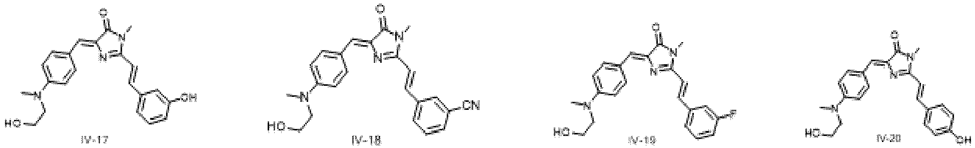

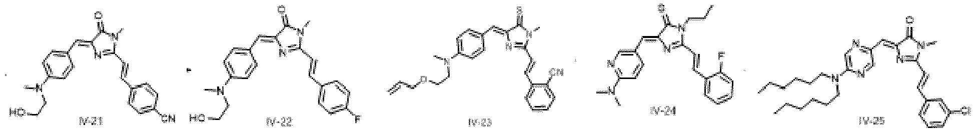

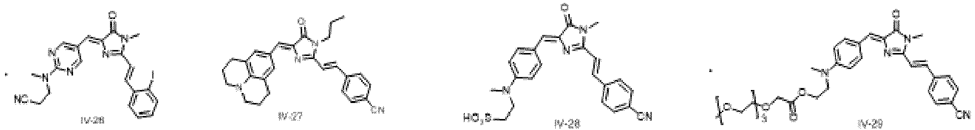

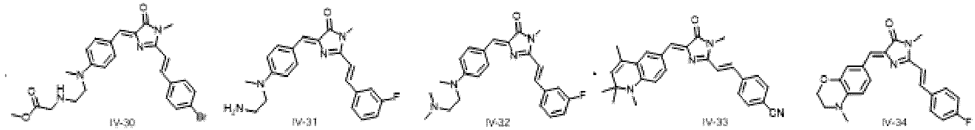

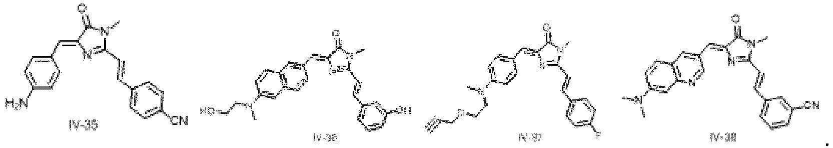

5. A method for preparing the fluorescent dye according to any one of claims 1 to 4, including a step of aldol condensation reaction between a compound of Formula (a) and a compound of Formula (b):

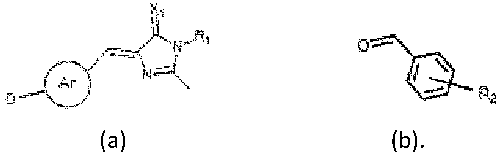

6. Uses of the fluorescent dye according to any one of claims 1 to 4 in viscosity testing, protein fluorescent labeling, nucleic acid fluorescent labeling, protein quantification or detection, or nucleic acid quantification or detection, wherein the uses are those other than for diagnostic methods of diseases.

7. Uses of the fluorescent dye according to any one of claims 1 to 4 in preparing reagents for viscosity testing, protein fluorescent labeling, nucleic acid fluorescent labeling, protein quantification or detection, or nucleic acid quantification or detection.

8. A fluorescent activated and lighted-up probe, comprising the fluorescent dye according to any one of claims 1 to 4.

9. Uses of the fluorescent activated and lighted-up probe according to claim 8 in protein fluorescent labeling, nucleic acid fluorescent labeling, protein quantification or detection, or nucleic acid quantification or detection, wherein the uses are those other than for diagnostic methods of diseases.

10. Uses of the fluorescent activated and lighted-up probe according to claim 8 in preparing reagents for protein fluorescent labeling, nucleic acid fluorescent labeling, protein quantification or detection, or nucleic acid quantification or detection.
